# EUROPEAN PATENT SPECIFICATION

(11) **EP 1 984 525 B1**
(45) Date of publication and mention of the grant of the patent: **26.06.2013**
(21) Application number: 07718079.2
(22) Date of filing: 19.01.2007
(51) Int. Cl.: G06F 19/18

(54) **METHOD FOR DETERMINING RESISTANCE OF HIV TO PROTEASE INHIBITOR TREATMENT**
VERFAHREN ZUR BESTIMMUNG DER RESISTENZ VON HIV GEGENÜBER EINER PROTEASEINHIBITOR-BEHANDLUNG
PROCÉDÉ PERMETTANT DE DÉTERMINER LA RÉSISTANCE DU VIH À UN TRAITEMENT PAR UN INHIBITEUR DE LA PROTÉASE

(30) Priority: 19.01.2006 US 760580 P; 29.03.2006 US 787550 P
(43) Date of publication of application: 29.10.2008
(73) Proprietor: Monogram BioSciences, Inc., South San Francisco, CA 94080 (US)
(72) Inventor: PARKIN, Neil, T., Belmont, CA 94002 (US); CHAPPEY, Colombe, San Francisco, CA 94110 (US)
(74) Representative: Weber, Martin
(86) International application number: PCT/US2007/001338
(87) International publication number: WO 2007/084618

(56) References cited:
- US-A1- 2005 214 749
- US-A1- 2005 233 312
- Valdez H. et al.,: "Tipranavir : predicting viral response" 14th international hiv drug resistance workshop (conference report) 7 June 2005 (2005-06-07), XP2566204 Retrieved from the Internet: URL:http://www.natap.org/2005/resistance/d rw_4.htm> [retrieved on 2010-01-02]
- JOHNON V.A.L ET AL.,: "Update of the drug resistance mutations in HIV-1: 2005" INTERNATIONAL AIDS SOCIETY-USA, vol. 13, no. 1, March 2005 (2005-03), pages 51-57, XP002566205
- DOYON ET AL: "Selection and characterization of HIV-1 showing reduced susceptibility to the non-peptidic protease inhibitor tipranavir" ANTIVIRAL RESEARCH, ELSEVIER BV, NL, vol. 68, no. 1, 1 October 2005 (2005-10-01), pages 27-35, XP005087834 ISSN: 0166-3542
- BAXTER J.D. ET AL.,: "genotypic changes in human immunodeficiency virus type 1 protease associated with reduced susceptibility and virologic response to protease inhibitor tipranavir" J. VIROLOGY, vol. 80, no. 21, November 2006 (2006-11), pages 10794-10801, XP002566206
- CROOM ET AL.: 'Tipranavir: A Ritonavir-Boosted Protease Inhibitor' DRUGS vol. 65, no. 12, 2005, pages 1669 - 1677, XP009128775

## Description

### 1. FIELD OF THE INVENTION

This invention relates to methods and devices for determining the susceptibility of a pathogenic virus to an anti-viral compound. In particular, this invention relates to methods and devices useful for the identification of HIV having altered, e.g., increased or decreased, susceptibility to protease inhibitors, such as, e.g., tipranavir ("TPV"), in a subject infected with HIV using genotypic information of the HIV.

### 2. BACKGROUND OF THE INVENTION

More than 60 million people have been infected with the human immunodeficiency virus ("HIV"), the causative agent of acquired immune deficiency syndrome ("AIDS"), since the early 1980s. *See* Lucas, 2002, Lepr Rev. 73(1):64-71. HIV/AIDS is now the leading cause of death in sub-Saharan Africa, and is the fourth biggest killer worldwide. At the end of 2001, an estimated 40 million people were living with HIV globally. *See* Norris, 2002, Radiol Technol. 73(4):339-363.

The goal of antiretroviral therapy drug treatment is to delay disease progression and prolong survival by achieving sustained suppression of viral replication. Current anti-HIV drugs target different stages of the HIV life cycle and a variety of enzymes essential for HIV's replication and/or survival. For example, certain drugs approved for AIDS therapy inhibit HIV replication by interfering with the enzymatic activities of either protease ("PR") or reverse transcriptase ("RT"). Among the approved drugs are nucleoside RT inhibitors ("NRTIs") such as AZT, ddI, ddC, d4T, 3TC, abacavir, nucleotide RT inhibitors such as tenofovir, non-nucleoside RT inhibitors ("NNRTIs") such as nevirapine, efavirenz, delavirdine and protease inhibitors ("PIs") such as saquinavir, ritonavir, indinavir, nelfinavir, amprenavir, lopinavir, atazanavir and tipranavir.

One consequence of the action of an anti-viral drug is that it can exert sufficient selective pressure on virus replication to select for drug-resistant mutants. Herrmann et al., 1977, Ann NY Acad Sci 284:632-637. With increasing drug exposure, the selective pressure on the replicating virus population increases to promote the more rapid emergence of drug resistant mutants.

With the inevitable emergence of drug resistance, strategies must be designed to optimize treatment in the face of resistant virus populations. Ascertaining the contribution of drug resistance to drug failure is difficult because patients that are likely to develop drug resistance are also likely to have other factors that predispose them to a poor prognosis. Richman, 1994, AIDS Res Hum Retroviruses 10:901-905. In addition, each patient typically harbors a diverse mixture of mutant strains of the virus with different mutant strains having different susceptibilities to anti-viral drugs.

Antiviral drug susceptibility assays for clinical HIV isolates are thus required to monitor the development of drug resistance during therapy. Ideally, assays that determine the drug susceptibility of HIV isolates should be rapid, reproducible, non-hazardous, applicable to all samples, and cost-effective. Two general approaches are now used for measuring resistance to anti-viral drugs. The first approach, called phenotypic testing, measures the susceptibility of virus taken from an infected person's virus to particular anti-viral drugs in an *in vitro* assay system. *See, e.g.,* Kellam & Larder, 1994, Antimicrob. Agents and Chemother 38:23-30; Petropoulos et al., 2000, Antimicrob. Agents Chemother 44:920-928; Fiertogs et al., 1998, Antimicrob. Agents Chemother 42(2):269-76. The second approach, genotypic testing, involves identifying the presence of mutations in the HIV nucleic acid that confer resistance to certain antiviral drugs in a patient infected with that virus.

Genotypic testing, in some aspects, promises certain advantages over phenotypic testing since the facilities necessary for genotypic testing are generally cheaper and less complex than those for phenotypic testing, and genotyping is typically less labor intensive to perform and results can be had in less time. However, in order to deduce the viral sensitivity from a given genotype, the effects on drug resistance of particular resistance mutations need to be known. An additional complication of genotypic assays is that the manual interpretation of such assays is difficult because a large number of drug resistance mutations interact in complex patterns.

Tipranavir is a non-peptidic protease inhibitor approved in 2005, which possesses broad antiviral activity against multiple protease inhibitor-resistant HIV-1. *See* Larder et al., 2000, AIDS 14:1943-48. Resistance to this protease inhibitor however has yet been well described. *See* Doyon et al., Antiviral Res. 2005, 68:27-35 describes the *in vitro* selection and characterization of HIV-1 variants having reduced susceptibility to tipranavir. Ten amino acid positions in the HIV protease were associated with reduced susceptibility to tipranavir. *See id.* A subsequent study found twenty one different mutations at sixteen positions in the HIV-1 protease that correlated with reduced tipranavir susceptibility or reduced response to tipranavir therapy. *See* Kohlbrenner *et al.,* meeting presentation entitled "Tipranavir Mutation Score Predicts ViroLogic Success in PI-Experienced HIV-Positive Adults," presented at HIV DART Conference (December 2004) ("the Kohlbrenner *et al.* study"). A crude algorithm that attempted to correlate the phenotypic resistance to tipranavir with the number of mutations observed at the sixteen identified positions, and therefore attempted to predict the effectiveness of tipranavir treatment, was postulated. *See id***.** According to the algorithm, a virus was susceptible to treatment with tipranavir if it had four or fewer mutations at the sixteen identified positions in its protease. If the number of mutations at these sixteen positions was four or more, then the virus was predicted to be resistant to tipranavir treatment. *See id.*

Efforts to date to use genotypic correlates of reduced susceptibility to predict the effectiveness of tipranavir are, at best, imperfect. An algorithm that can more accurately predict whether a given anti-viral drug or combination of drugs would be effective in treating a given patient would save time and money by identifying drugs and drug combinations that are not likely to succeed before they are administered to the patient. More importantly, it would improve the quality of life of the patient by sparing him or her the trauma of treatment with potent toxins that result in no improvement with respect to his or her HIV infection. Therefore, an urgent need exists for a more accurate algorithm for predicting whether tipranavir would be effective for treating a particular patient.

### 3. SUMMARY OF THE INVENTION

The present invention provides methods and compositions for developing and using algorithms for determining the effectiveness of an anti-viral therapy or combination of therapies. The algorithms are based on an analysis of paired phenotypic and genotypic data guided by phenotypic cut-offs (the point at which resistance to a therapy begins and sensitivity ends). The algorithms significantly improve the quality of life of a patient by accurately predicting whether a given anti-viral drug would be effective in treating the patient, thereby sparing him or her the trauma of treatment with potent toxins that result in no improvement in his or her HIV infection.

In one aspect, the present invention provides algorithms that allow one to provide a patient with an effective treatment regimen by predicting whether an infected individual will respond to treatment with an anti-viral agent or combination of agents, thereby allowing an effective treatment regimen to be designed without subjecting the patient to unnecessary side effects. Also, by avoiding the administration of ineffective drugs, considerable time and money is saved.

In another aspect, the present invention provides methods for determining the susceptibility of a virus to an anti-viral treatment, comprising detecting, in the viral genome or viral enzymes, the presence or absence of mutations associated with reduced susceptibility to the anti-viral treatment.

In another aspect, the present invention provides methods for determining the effectiveness of an anti-viral treatment of an individual infected with a virus, comprising detecting, in a sample from said individual, the presence or absence of mutations in the viral genome or proteins associated with reduced susceptibility to the anti-viral treatment.

The present invention also provides methods of monitoring the clinical progression of viral infection in individuals receiving an anti-viral treatment by determining, as described above, the effectiveness of the same or a different anti-viral treatment.

In one aspect, the invention provides a method for determining whether an HIV-1 is likely to be exhibit reduced susceptibility to treatment with a protease inhibitor, such as tipranavir, comprising: detecting, in a gene encoding a protease of the HIV-1, the presence or absence of the HIV-1 protease mutations listed in Table 3, each of said mutations being associated with a weighting factor as shown in Table 3; and summing the weighting factors for each of the mutations that are detected to compute a mutation score for the HIV-1. Said HIV-1 is likely to exhibit reduced susceptibility to treatment with said protease inhibitor if said total score is equal to or greater than a cut-off score and the HIV-1 is likely to be susceptible to treatment with said protease inhibitor if said total score is less than said cut-off score. In certain embodiments, the HIV-1 is partially resistant to said treatment. In certain embodiments, the HIV-1 is resistant to said treatment. In one embodiment, the cut-off score is 4. In another embodiment, the cut-off score is 5. In another embodiment, the cut-off score is 6. In another embodiment, the cut-off score is 7.

In another aspect, the invention provides a method for determining whether an HIV-1 infecting an individual is likely to exhibit reduced susceptibility to treatment with a protease inhibitor, such as tipranavir, comprising: detecting, in a sample from the individual, the presence or absence of one or more of the HIV-1 protease mutations listed in Table 3, each of said mutations being associated with a weighting factor as shown in Table 3; and summing said weighting factors that are detected in step (a) to compute a mutation score for the HIV-1. Said HIV-1 is likely to exhibit reduced susceptibility to treatment with said protease inhibitor if said total score is equal to or greater than a cut-off score and said HIV-1 is likely to be susceptible to treatment with said protease inhibitor if said total score is less than said cut-off score. In certain embodiments, the HIV-1 is partially resistant to said treatment. In certain embodiments, the HIV-1 is resistant to said treatment. In one embodiment, the cut-off score is 4. In another embodiment, the cut-off score is 5. In another embodiment, the cut-off score is 6. In another embodiment, the cut-off score is 7.

In another aspect, the invention provides a method for determining whether an HIV-1 is likely to be exhibit reduced susceptibility to treatment with a protease inhibitor; such as tipranavir, comprising: detecting, in a gene encoding a protease of the HIV-1, the presence or absence of one or more of the HIV-1 protease mutations listed in Table 9, each of said mutations being associated with a weighting factor as shown in Table 9; and summing the weighting factors for each of the mutations that are detected to compute a mutation score for the HIV-1. Said HIV-1 is likely to exhibit reduced susceptibility to treatment with said protease inhibitor if said total score is equal to or greater than a cut-off score and the HIV-1 is likely to be susceptible to treatment with said protease inhibitor if said total score is less than said cut-off score. In certain embodiments, the HIV-1 is partially resistant to said treatment. In certain embodiments, the HIV-1 is resistant to said treatment. In one embodiment, the cut-off score is 4. In another embodiment, the cut-off score is 5. In another embodiment, the cut-off score is 6. In another embodiment, the cut-off score is 7. In another aspect, the invention provides a method for determining whether an HIV-1 infecting an individual is likely to exhibit reduced susceptibility to treatment with a protease inhibitor, such as tipranavir, comprising: detecting, in a sample from the individual, the presence or absence of one or more of the HIV-1 protease mutations listed in Table 9, each of said mutations being associated with a weighting factor as shown in Table 9; and summing said weighting factors that are detected in step (a) to compute a mutation score for the HIV-1. Said HIV-1 is likely to exhibit reduced susceptibility to treatment with said protease inhibitor if said total score is equal to or greater than a cut-off score and said HIV-1 is likely to be susceptible to treatment with said protease inhibitor if said total score is less than said cut-off score. In certain embodiments, the HIV-1 is partially resistant to said treatment. In certain embodiments, the HIV-1 is resistant to said treatment. In one embodiment, the cut-off score is 4. In another embodiment, the cut-off score is 5. In another embodiment, the cut-off score is 6. In another embodiment, the cut-off score is 7.

In another aspect, the invention provides a method for determining whether a HIV-1 has an increased likelihood of having reduced susceptibility to treatment with a protease inhibitor, comprising detecting in the protease of said HIV or in a nucleic acid of said HIV that encodes the protease; the presence or absence of a mutation associated with reduced susceptibility to treatment with said protease inhibitor at amino acid position 10, 11, 32, 36, 46, 55, 60, 71, 73, 84, 89 and/or 90 of the amino acid sequence of said protease, wherein the presence of said mutation indicates that the HIV has an increased likelihood of having a reduced susceptibility to treatment with the protease inhibitor. In certain embodiments, the HIV-1 is partially resistant to said treatment. In certain embodiments, the HIV-1 is resistant to said treatment. In some embodiments, the protease inhibitor is tipranavir. The presence of the mutations associated with reduced susceptibility to a protease inhibitor can be detected according to any method known to one of skill in the art without limitation. Methods for detecting such mutations are described below.

In some embodiments, the mutation associated with reduced susceptibility to treatment with said protease inhibitor is selected from the group consisting of: L10I, V11L, V32I, M46I, K55R, D60E, A71L, G73T, I84V, L89V and L90M.

In another aspect, the invention provides a method for determining whether an individual infected with HIV has an increased likelihood of having reduced susceptibility to treatment with a protease inhibitor, comprising detecting, in a sample from said individual, the presence or absence of a mutation associated with reduced susceptibility to treatment with said protease inhibitor at amino acid position 10, 11, 32, 36, 46, 55, 60, 71, 73, 84, 89 and/or 90 of the amino acid sequence of the protease of the HIV, wherein the presence of said mutation indicates that the individual has an increased likelihood of having a reduced susceptibility to treatment with the protease inhibitor. In some embodiments, the protease inhibitor is tipranavir. The presence of the mutations associated with reduced susceptibility to a protease inhibitor can be detected according to any method known to one of skill in the art without limitation. Methods for detecting such mutations are described below.

In some embodiments, the mutation associated with reduced susceptibility to treatment with said protease inhibitor is selected from the group consisting of: L10I, V11L, V32I, M46I, K55R, D60E, A71L, G73T, I84V, L89V and L90M.

In another aspect, the invention provides a method for determining whether an HIV-1 has an increased likelihood of having an increased susceptibility to treatment with a protease inhibitor, comprising detecting in the protease of said HIV or in a nucleic acid of said HIV that encodes the protease, the presence or absence of a mutation associated with increased susceptibility to treatment with said protease inhibitor at amino acid position 10, 24, 30, 50, 54, 76, 82 and/or 88 of the amino acid sequence of said protease, wherein the presence of said mutation indicates that the HIV has an increased likelihood of having increased susceptibility to treatment with the protease inhibitor. In some embodiments, the protease inhibitor is tipranavir. The presence of the mutations associated with altered susceptibility to a protease inhibitor can be detected according to any method known to one of skill in the art without limitation. Methods for detecting such mutations are described below.

In some embodiments, the mutation associated with increased susceptibility to treatment with said protease inhibitor is selected from the group consisting of: L10F, L24I, D30N, I50L/V, I54L, L76V, V82I and N88D.

In another aspect, the invention provides a method for determining whether an HIV infecting an individual has an increased likelihood of having an increased susceptibility to treatment with a protease inhibitor, comprising detecting, in a sample from said individual, the presence or absence of a mutation associated with increased susceptibility to treatment with said protease inhibitor at amino acid position 10, 24, 30, 50, 54, 76, 82 and/or 88 of the amino acid sequence of the protease of the HIV, wherein the presence of said mutation indicates that the HIV-1 has an increased likelihood of having increased susceptibility to treatment with the protease inhibitor. In some embodiments, the protease inhibitor is tipranavir. The presence of the mutations associated with altered susceptibility to a protease inhibitor can be detected according to any method known to one of skill in the art without limitation. Methods for detecting such mutations are described below.

In some embodiments, the mutation associated with increased susceptibility to treatment with said protease inhibitor is selected from the group consisting of: L10F, L24I, D30N, I50L/V, I54L, L76V, V82I and N88D.

In one aspect, the present invention provides a computer implemented method for determining whether an HIV-1 is likely to be resistant to a protease inhibitor, comprising inputting to a computer-readable medium genotypic data from an HIV-1 protease of the HIV-1; comparing the genotypic data to a database that comprises a set of mutations, each of said mutations being associated with a weighting factor as shown in Table 3; and calculating a mutation score by summing the weighting factors associated with the mutation(s) present in the genotype of the HIV-1, wherein if the mutation score is equal to or greater than a cutoff score, the HIV-1 is determined to exhibit reduced susceptibility to tipranavir. In certain embodiments, the HIV-1 is partially resistant to tipranavir. In certain embodiments, the HIV-1 is resistant to tipranavir.

In certain embodiments, the computer implemented method further comprises displaying whether or not the HIV-1 is determined to be resistant to tipranavir. For example, the result may be displayed on a tangible medium such as paper or other form of printout or on a computer screen, or other tangible media without limitation.

In another aspect, the present invention provides a computer implemented method for determining whether an HIV-1 is likely to be resistant to a protease inhibitor, comprising inputting to a computer-readable medium genotypic data from an HIV-1 protease of the HIV-1; comparing the genotypic data to a database that comprises a set of mutations, each of said mutations being associated with a weighting factor as shown in Table 9; and calculating a mutation score by summing the weighting factors associated with the mutation(s) present in the genotype of the HIV-1, wherein if the mutation score is equal to or greater than a cutoff score, the HIV-1 is determined to exhibit reduced susceptibility to tipranavir. In certain embodiments, the HIV-1 is partially resistant to tipranavir. In certain embodiments, the HIV-1 is resistant to tipranavir.

In certain embodiments, the computer implemented method further comprises displaying whether or not the HIV-1 is determined to be resistant to tipranavir. For example, the result may be displayed on a tangible medium such as paper or other form of printout or on a computer screen, or other tangible media without limitation.

In another aspect, the invention provides an article of manufacture that comprises computer-readable instructions for performing a computer implemented method of the invention. For example, the article of manufacture can be a floppy disk, CD, DVD, magnetic tape, and so forth, without limitation.

In another aspect, the present invention provides a computer system that is configured to perform a computer implemented method of the invention.

In another aspect, the present invention provides a computer-readable medium comprising a computer program that determines whether an HIV-1 infecting a subject is resistant to a protease inhibitor, such as, *e*.*g*., tipranavir, comprising a computer code that receives input corresponding to a genotype of a HIV-1 nucleic acid encoding HIV-1 protease obtained from HIV-1 infecting the subject; a computer code that performs a comparison to determine if mutations listed in Table 3 are present; a computer code that calculates a mutation score based on the number of mutations present; a computer code that determines whether the mutation score is equal to or greater than a cutoff score, wherein the HIV-1 is determined to be resistant to tipranavir if the mutation score is equal to or greater than the cutoff score; and a computer code that conveys a result representing whether or not the HIV-1 is determined to have a reduced susceptibility to tipranavir to an output device.

In another aspect, the present invention provides a computer-readable medium comprising a computer program that determines whether an HIV-1 infecting a subject is resistant to a protease inhibitor, such as, *e*.*g*., tipranavir, comprising a computer code that receives input corresponding to a genotype of a HIV-1 nucleic acid encoding HIV-1 protease obtained from HIV-1 infecting the subject; a computer code that performs a comparison to determine if mutations listed in Table 9 are present; a computer code that calculates a mutation score based on the number of mutations present; a computer code that determines whether the mutation score is equal to or greater than a cutoff score, wherein the HIV-1 is determined to be resistant to tipranavir if the mutation score is equal to or greater than the cutoff score; and a computer code that conveys a result representing whether or not the HIV-1 is determined to have a reduced susceptibility to tipranavir to an output device.

In certain embodiments, the output device is a printer. In certain embodiments, the output device is a computer display, *e*.*g*., a flat panel display or a CRT monitor.

In another aspect, the invention provides a tangible medium comprising the result conveyed to the output device by the computer program product described above. In certain embodiments, the tangible medium is a printout. In other embodiments, the tangible medium is a CD or DVD.

In another aspect, the invention provides a computer-readable medium comprising data indicating whether an HIV-1 is resistant to a protease inhibitor, such as, *e.g.,* tipranavir, and computer-readable instructions for performing a method of the invention as described herein. In certain embodiments, the tangible medium is a floppy disk, CD, DVD, magnetic tape, fixed disk drive, iPod™, and the like.

### 4. BRIEF DESCRIPTION OF THE FIGURES

FIG. 1 is a diagrammatic representation of mutations associated with reduced susceptibility to tipranavir identified in the Kohlbrenner *et al.* study.

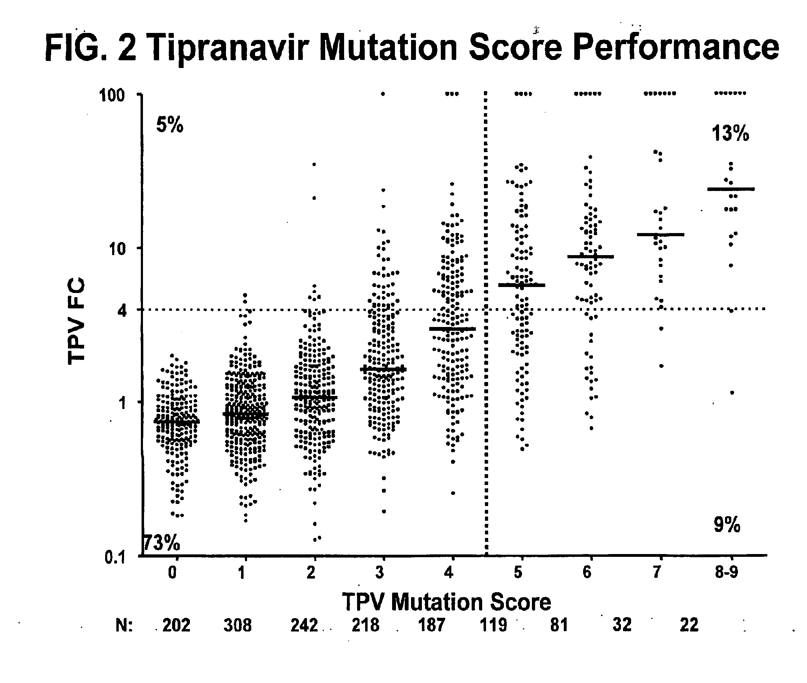

FIG. 2 is a scatter plot that shows the susceptibility to tipranavir (Log tipranavir fold change) of samples obtained from a data set with 1411 patient samples as a function of the number of the mutations in the protease associated with reduced susceptibility to tipranavir. The genotypic cutoff score is 5, *i.e.,* an HIV-1 is defined as having a reduced susceptibility to tipranavir genotype if its total score is 5 or greater and a sensitive genotype if its total score is less than 5. The phenotypic cutoff score is 4, *i.e.,* an HIV-1 is defined as having a reduced susceptibility to tipranavir phenotype if an IC₅₀ fold change is greater than 4 for tipranavir, and a sensitive phenotype if its total score is less than 4.

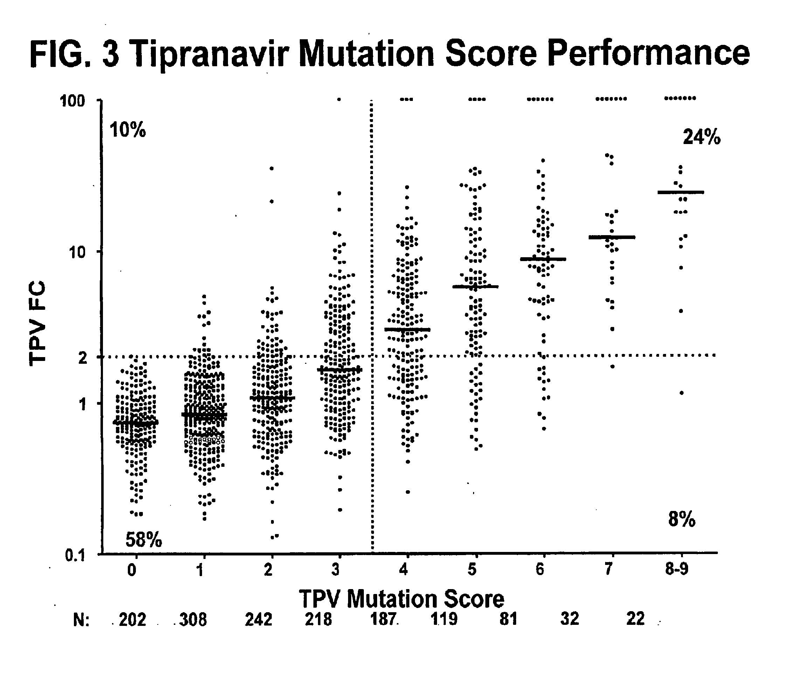

FIG. 3 is a scatter plot that shows the susceptibility to tipranavir (Log tipranavir fold change) of samples obtained from a data set with 1411 patient samples as a function of the number of the mutations in the protease associated with reduced susceptibility to tipranavir. The genotypic cutoff score is 4, *i.e.,* an HIV-1 is defined as having a reduced susceptibility to tipranavir genotype if its total score is 4 or greater and a sensitive genotype if its total score is less than 4. The phenotypic cutoff score is 2, *i.e.,* an HIV-1 is defined as having a reduced susceptibility to tipranavir phenotype if an IC₅₀ fold change is greater than 2 for tipranavir, and a sensitive phenotype if its total score is less than 2.

FIG. 4 is a scatter plot that shows a ratio of tipranavir susceptibility (fold change) of samples obtained from a data set with 1411 patient samples to the median fold change of samples with the same tipranavir mutation score, as a function of the number of the resistance-associated mutation in the protease. The median IC₅₀ fold change for tipranavir for samples in each category defined by mutation score was calculated. Samples were grouped based on measured tipranavir fold change being higher ("H") or lower ("L") than the median for each group.

FIG. 5 shows that the correlation between tipranavir fold change and mutation score calculated using the algorithm in the Kohlbrenner *et al.* study has a linear regression coefficient of 0.51.

FIG. 6 is a scatter plot showing that the correlation between tipranavir fold change and mutation score calculated using the algorithm in the Kohlbrenner *et al.* study has a linear regression coefficient of 0.51.

FIG. 7 shows that the correlation between tipranavir fold change and mutation score calculated using the algorithm of the present invention has a linear regression coefficient of 0.66.

FIG. 8 is a scatter plot showing that the correlation between tipranavir fold change and mutation score calculated using the algorithm of the present invention has a linear regression coefficient of 0.66.

FIG. 9 is a scatter plot that shows application of the old algorithm to a naïve dataset with 1845 patient samples. The genotypic cutoff score is 4, *i.e.,* an HIV-1 is defined as having a reduced susceptibility to tipranavir genotype if its total score is 4 or greater and a sensitive genotype if its total score is less than 4. The phenotypic cutoff score is 2, *i.e.,* an HIV-1 is defined as having a reduced susceptibility to tipranavir phenotype if an IC₅₀ fold change is greater than 2 for tipranavir, and a sensitive phenotype if its total score is less than 2.

FIG. 10 is a scatter plot that shows application of the new algorithm to a naïve dataset with 1845 patient samples. The genotypic cutoff score is 4, *i.e.,* an HIV-1 is defined as having a reduced susceptibility to tipranavir genotype if its total score is 4 or greater and a sensitive genotype if its total score is less than 4. The phenotypic cutoff score is 2, *i*.*e*., an HIV-1 is defined as having a reduced susceptibility to tipranavir phenotype if an IC₅₀ fold change is greater than 2 for tipranavir, and a sensitive phenotype if its total score is less than 2.

FIG. 11 is a scatter plot that shows the susceptibility to tipranavir (Log tipranavir fold change) of samples obtained from a data set with 4492 patient samples as a function of mutation score of the sample calculated with the old algorithm.

FIGS. 12A-C present a regression tree showing the predictive ability of particular mutations for TPV resistance or susceptibility.

FIGS. 13A-B present a classification tree showing the predictive ability of particular mutations for TPV resistance or susceptibility.

FIG. 14 is a scatter plot that shows the susceptibility to tipranavir (Log tipranavir fold change) of samples obtained from a data set with 3880 patient samples as a function of the mutation score of the sample calculated with the adjusted algorithm.

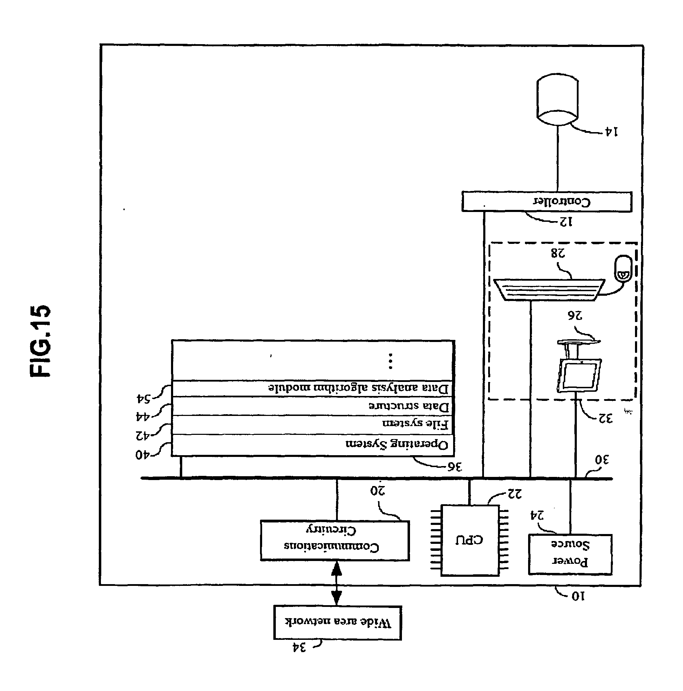

FIG. 15 illustrates a computer system in accordance with an embodiment of the present invention.

### 5. DETAILED DESCRIPTION OF THE INVENTION

The present invention provides methods and devices for identifying HIV-1 populations that have altered susceptibility to a protease inhibitor ("PI"). In certain aspects, the methods comprise use of a genotype interpretation algorithm. The present invention also provides methods for determining the susceptibility of a virus to an anti-viral treatment, methods for determining the effectiveness of an anti-viral treatment of an individual infected with a virus, and methods of monitoring the clinical progression of viral infection in individuals receiving anti-viral treatment.

The algorithms utilized in the methods of the invention have been developed by analysis and evaluation of the genotypes of a large dataset HIV-1 of known phenotypes to determine sets of protease mutations and combinations of these mutations that confer altered susceptibility to protease inhibitors. The following describes generally methods of generating genotype interpretation algorithms for the purpose of identifying drug resistant viruses

### 5.1 Abbreviations

"HIV" is an abbreviation for human immunodeficiency virus. "PR" is an abbreviation for protease. "PI" is an abbreviation for protease inhibitor. "PCR" is an abbreviation for polymerase chain reaction. "TPV" is an abbreviation for tipranavir. "FC" is an abbreviation for fold change. "GT-R or Gr," "GT-S or Gs," "PT-R or Pr" and "PT-S or Ps" are abbreviations for genotypically resistant or partially resistant, genotypically susceptible, phenotypically having a reduced susceptibility and phenotypically susceptible, respectively.

The amino acid notations used herein for the twenty genetically encoded L-amino acids are conventional and are as follows:

| **Amino Acid** | **One-Letter Abbreviation** | **Three Letter Abbreviation** |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

Unless noted otherwise, when polypeptide sequences are presented as a series of one-letter and/or three-letter abbreviations, the sequences are presented in the N-terminus to C-terminus direction, in accordance with common practice.

Substituted or mutant amino acids in HIV-1 protease positions are represented herein in an abbreviated fashion such as "M184I/L/V," where "M" is single-letter representation of the non-mutant reference amino acid methionine at position "184" of HIV-1 protease , and "I," "L" and "V" represent single-letter representations of possible mutant amino acids that may be substituted for M at position 36 in the protease.

### 5.2 Definitions -

As used herein, "genotypic data" are data about the genotype of, for example, a virus. Examples of genotypic data include, but are not limited to, the nucleotide or amino acid sequence of a virus, a part of a virus, a viral gene, a part of a viral gene, or the identity of one or more nucleotides or amino acid residues in a viral nucleic acid or protein.

A "genotypic assay" is a test that determines a genetic sequence of an organism, a part of an organism, a gene or a part of a gene. Such assays are frequently performed in HIV to establish whether certain mutations are associated with drug resistance are present.

A "phenotypic assay" is a test that measures the sensitivity of a virus (such as HIV) to a specific anti-viral agent.

"Susceptibility" refers to a virus' response to a particular drug. A virus that is less susceptible or has decreased susceptibility to a drug is less sensitive or more resistant to the drug. A virus that has increased or enhanced or greater susceptibility to a drug has an increased sensitivity or decreased resistance to the drug.

Phenotypic drug susceptibility is measured as the concentration of drug required to inhibit virus replication by 50% (IC₅₀). As used herein, a "fold change" or "FC". is the ratio of a viral variant IC₅₀ divided by the IC₅₀ of a reference HIV. An FC of 1.0 indicates that the viral variant exhibits the same degree of drug susceptibility as the reference virus.

For drugs where sufficient clinical outcome data have been gathered, it is possible to define a clinical threshold or cutoff value. A clinical threshold or cutoff value defines the point above which the utility of a given drug begins to decline based on virologic response data from clinical trials. It represents a point of increasing resistance and decreasing sensitivity of the HIV to a particular drug. The cutoff value is different for different anti-viral agents. Clinical cutoff values are determined in clinical trials by evaluating resistance and outcome data. Drug susceptibility is measured at treatment initiation. Treatment response, such as change in viral load, is monitored at predetermined time points through the course of the treatment. The drug susceptibility is correlated with treatment response and the clinical cutoff value is determined by resistance levels associated with treatment failure (statistical analysis of overall trial results).

"ICₙ" refers to Inhibitory Concentration. It is the concentration of drug in the patient's blood or *in vitro* needed to suppress the reproduction of a disease-causing microorganism (such as HIV) by n %. Thus, "IC₅₀" refers to the concentration of an anti-viral agent at which virus replication is inhibited by 50% of the level observed in the absence of the drug. "Patient IC₅₀" refers to the drug concentration required to inhibit replication of the virus from a patient by 50% and "reference IC₅₀" refers to the drug concentration required to inhibit replication of a reference or wild-type virus by 50%. Similarly, "IC₉₀" refers to the concentration of an anti-viral agent at which 90% of virus replication is inhibited.

A "fold change" is a numeric comparison of the drug susceptibility of a patient virus and a drug-sensitive reference virus. It is the ratio of the Patient IC₅₀ to the drug-sensitive reference IC₅₀, *i.e*., Patient IC₅₀/Reference IC₅₀ = Fold Change ("FC"). A fold change of 1.0 indicates that the patient virus exhibits the same degree of drug susceptibility as the drug-sensitive reference virus. A fold change less than 1 indicates the patient virus is more sensitive than the drug-sensitive reference virus. A fold change greater than 1 indicates the patient virus is less susceptible than the drug-sensitive reference virus. A fold change equal to or greater than the clinical cutoff value means the patient virus has a lower probability of response to that drug. A fold change less than the clinical cutoff value means the patient virus is sensitive to that drug.

"Tipranavir fold change" refers to the ratio of the IC₅₀ of tipranavir against the HIV from the patient plasma sample to the IC₅₀ for tipranavir against the NL4-3 (GenBank Accession No. AF324493) reference viral strain.

A virus is "sensitive" to tipranavir if it has a tipranavir fold change less than the "lower clinical cutoff." In some embodiments, a virus is sensitive to tipranavir if it has a tipranavir fold change less than 4. In some embodiments, a virus is sensitive to tipranavir if it has a tipranavir fold change less than 2.

A virus is "resistant" to tipranavir if it has a tipranavir fold change of greater than the "upper clinical cutoff." In some embodiments, a virus is resistant to tipranavir if it has a tipranavir fold change greater than 10. In some embodiments, a virus is resistant to tipranavir if it has a tipranavir fold change greater than 15.

A virus is "partially resistant" to tipranavir if it has a tipranavir fold change of greater than the "lower clinical cutoff" but lower than the "upper clinical cutoff'. In some embodiments, a virus is partially resistant to tipranavir if it has a tipranavir fold change between 2 and 10. In some embodiments, a virus is partially resistant to tipranavir if it has a tipranavir fold change between 4 and 10. In some embodiments, a virus is partially resistant to tipranavir if it has a tipranavir fold change between 2 and 15. In some embodiments, a virus is partially resistant to tipranavir if it has a tipranavir fold change between 4 and 15.

The term "mutation" refers to any change in the nucleic acid sequence of a given HIV, or in the polypeptide sequence of the proteins of a given HIV, as compared to a reference HIV.

The terms "peptide," "polypeptide" and "protein" are used interchangeably throughout.

The terms "polynucleotide," "oligonucleotide" and "nucleic acid" are used interchangeably throughout.

The term "concordance" as used herein, means that a genotype from an HIV sample categorized as GT-R or GT-S according to an algorithm matches the phenotype (PT-R or PT-S) of that HIV sample.

The term "discordance" as used herein, means that a genotype from an HIV sample categorized as GT-R or GT-S according to an algorithm does not match the phenotype of the that HIV sample. Discordance samples include both false negatives (GT-S, PT-R) and false positive (GT-R, PT-S) identifications.

The methods and devices of the present invention arise, in part, out of the creation of an algorithm that predicts HIV altered susceptibility to tipranavir based on a HIV's genotype. The methods and devices disclosed herein significantly increase the availability of information to health care professionals and HIV infected persons for making informed choices regarding tipranavir drug therapy.

### 5.3 Determining Susceptibility to the Anti-Viral Treatment

In one aspect, the present invention provides a method for determining a virus' susceptibility to anti-viral treatment. Mutations associated with altered susceptibility to drugs can be identified and correlated with altered susceptibility of a virus to an anti-viral treatment as described in Sections 5.4-5.5 below. The presence of such a mutation in a virus can be detected by any means known in the art, e.g., as discussed in Section 5.4 below. The presence of a mutation associated with altered susceptibility to drugs in the virus can indicate that the virus has an increased likelihood of having either a reduced or an increased susceptibility for the anti-viral treatment. In one embodiment, the virus is human immunodeficiency virus (HIV). In another embodiment, the virus is human immunodeficiency virus type-1 (HIV-1). In another embodiment, the anti-viral treatment is a protease inhibitor. Examples of protease inhibitors include, but are not limited to, saquinavir, ritonavir, indinavir, nelfinavir, amprenavir and tipranavir. In one embodiment, the protease inhibitor is tipranavir.

In another aspect, the invention provides a method for determining whether a HIV-1 has an increased likelihood of having a reduced susceptibility to treatment with a protease inhibitor, such as tipranavir, comprising detecting in the protease of said HIV or in a nucleic acid of said HIV that encodes the protease, the presence or absence of a mutation associated with reduced susceptibility to treatment with said protease inhibitor at amino acid position 10, 11, 32, 36, 46, 55, 60, 71, 73, 84, 89 and/or 90 of the amino acid sequence of said protease, wherein the presence of said mutation indicates that the HIV has an increased likelihood of having a reduced susceptibility to treatment with the protease inhibitor. The presence of the mutations associated with reduced susceptibility to a protease inhibitor can be detected according to any method known to one of skill in the art without limitation. Methods for detecting such mutations are described below.

In some embodiments, the mutation associated with reduced susceptibility to treatment with said protease inhibitor is selected from the group consisting of: L1I, V11L, V32I, M46I, K55R, D60E, A71L, G73T, I84V, L89V and L90M.

In another aspect, the invention provides a method for determining whether an HIV-1 infecting an individual has an increased likelihood of having a reduced susceptibility to treatment with a protease inhibitor, such as tipranavir, comprising detecting, in a sample from said individual, the presence or absence of a mutation associated with reduced susceptibility to treatment with said protease inhibitor at amino acid position 10, 11, 32, 36, 46, 55, 60, 71, 73, 84, 89 and/or 90 of the amino acid sequence of the protease of the HIV, wherein the presence of said mutation indicates that the HIV-1 has an increased likelihood of having a reduced susceptibility to treatment with the protease inhibitor. The presence of the mutations associated with reduced susceptibility to a protease inhibitor can be detected according to any method known to one of skill in the art without limitation. Methods for detecting such mutations are described herein.

In some embodiments, the mutation associated with reduced susceptibility to treatment with said protease inhibitor is selected from the group consisting of: L10I, V11L, V32I, M46I, K55R, D60E, A71L, G73T, I84V, L89V and L90M.

In another aspect, the invention provides a method for determining whether an HIV-1 has an increased likelihood of having an increased susceptibility to treatment with a protease inhibitor, such as tipranavir, comprising detecting in the protease of said HIV or in a nucleic acid of said HIV that encodes the protease, the presence or absence of a mutation associated with increased susceptibility to treatment with said protease inhibitor at amino acid position 10, 24, 30, 50, 54, 76, 82 and/or 88 of the amino acid sequence of said protease, wherein the presence of said mutation indicates that the HIV has an increased likelihood of having increased susceptibility to treatment with the protease inhibitor. The presence of the mutations associated with altered susceptibility to a protease inhibitor can be detected according to any method known to one of skill in the art without limitation. Methods for detecting such mutations are described below.

In some embodiments, the mutation associated with increased susceptibility to treatment with said protease inhibitor is selected from the group consisting of: L10F, L24I, D30N, I50L/V, I54L, L76V, V82I and N88D.

In another aspect, the invention provides a method for determining whether an HIV-1 infecting an individual has an increased likelihood of having an increased susceptibility to treatment with a protease inhibitor, such as tipranavir, comprising detecting, in a sample from said individual, the presence or absence of a mutation associated with increased susceptibility to treatment with said protease inhibitor at amino acid position 10, 24, 30, 50, 54, 76, 82 or 88 of the amino acid sequence of the protease of the HIV, wherein the presence of said mutation indicates that the HIV-1 has an increased likelihood of having increased susceptibility to treatment with the protease inhibitor. The presence of the mutations associated with altered susceptibility to a protease inhibitor can be detected according to any method known to one of skill in the art without limitation. Methods for detecting such mutations are described below.

In some embodiments, the mutation associated with increased susceptibility to treatment with said protease inhibitor is selected from the group consisting of: L10F, L24I, D30N, I50L/V, I54L, L76V, V82I and N88D.

### 5.4 Detecting the Presence or Absence of Mutations in a Virus

The presence or absence of a viral mutation according to the present invention can be detected by any means known in the art for detecting a mutation. Typically mutations of interest are those identified to confer resistance to a particular antiviral drug or combination of drugs, either existing alone or in a combination with other mutations. Thus, the mutation can be detected in the viral gene that encodes a particular protein, or in the protein itself, *i*.*e*., in the amino acid sequence of the protein.

In one embodiment, the mutation is in the viral nucleic acid. Such a mutation can be in, for example, a gene encoding a viral protein, in a *cis* or *trans* acting regulatory sequence of a gene encoding a viral protein, an intergenic sequence, or an intron sequence. The mutation can affect any aspect of the structure, function, replication or environment of the virus that changes its susceptibility to an anti-viral treatment. In one embodiment, the mutation is in a gene encoding a viral protein that is the target of an anti-viral treatment.

In another embodiment, the mutation is in an HIV-1 nucleic acid encoding a protease. For example, the mutation can be any mutation in codon 10, 11, 13, 20, 24, 30, 32, 33, 35, 36, 43,46,47, 50, 54, 58, 69, 71, 73, 74, 76, 82, 83, 84, 89 or 90. In one embodiment, the mutation in the nucleic acid encoding HIV-1 protease is selected from the group consisting of L10I/V, I13V, K20M/V/R, L24I, D30N, V32I, L33F, E35G, M36I, K43T, M46I/L, I47V, 150L, I54A/L/M/V, Q58E, H69K, A71L, G73T, T74P, L76V, V82I/L/T, N83D, I84V, L89V, and L90M.

In certain embodiments, the mutation in an HIV-1 nucleic acid encoding HIV-1 protease correlates with reduced susceptibility to tipranavir as described herein. In certain embodiments, the mutation correlates with partial resistance to tipranavir. In certain embodiments, the mutation correlates with resistance to tipranavir. In certain embodiments, the mutation in an HIV-1 nucleic acid encoding HIV-1 protease correlates with increased susceptibility to tipranavir as described herein.

A mutation within a viral gene can be detected by utilizing any method known by one of skill in the art without limitation. Viral DNA or RNA can be used as the starting point for such assay techniques, and may be isolated according to standard procedures which are well known to those of skill in the art.

The detection of a mutation in specific nucleic acid sequences, such as in a particular region of a viral gene, can be accomplished by a variety of methods including, but not limited to, restriction-fragment-length-polymorphism detection based on allele-specific restriction-endonuclease cleavage (Kan and Dozy, 1978, Lancet ii:910-912), mismatch-repair detection (Faham and Cox, 1995, Genome Res 5:474-482), binding of MutS protein (Wagner et al., 1995, Nucl Acids Res 23:3944-3948), denaturing-gradient gel electrophoresis (Fisher et al., 1983, Proc. Natl. Acad. Sci. U.S.A. 80:1579-83), single-strand-conformation-polymorphism detection (Orita et al., 1983, Genomics 5:874-879), RNAase cleavage at mismatched base-pairs (Myers et al., 1985, Science 230:1242), chemical (Cotton et al., 1988, Proc. Natl. Acad. Sci. U.S.A. 85:4397-4401) or enzymatic (Youil et al., 1995, Proc. Natl. Acad. Sci. U.S.A. 92:87-91) cleavage ofheteroduplex DNA, methods based on oligonucleotide-specific primer extension (Syvänen et al., 1990, Genomics 8:684-692), genetic bit analysis (Nikiforov et al., 1994, Nucl Acids Res 22:4167-4175), oligonucleotide-ligation assay (Landegren et al., 1988, Science 241:1077), oligonucleotide-specific ligation chain reaction ("LCR") (Barrany, 1991, Proc. Natl. Acad. Sci. U.S.A. 88:189-193), gap-LCR (Abravaya et al., 1995, Nucl Acids Res 23:675-682), radioactive or fluorescent DNA sequencing using standard procedures well known in the art, and peptide nucleic acid (PNA) assays (Orum et al., 1993, Nucl. Acids Res. 21:5332-5356; Thiede et al., 1996, Nucl. Acids Res. 24:983-984).

In addition, viral DNA or RNA may be used in hybridization or amplification assays to detect abnormalities involving gene structure, including point mutations, insertions, deletions and genomic rearrangements. Such assays may include, but are not limited to, Southern analyses (Southern, 1975, J. Mol. Biol. 98:503-517), single stranded conformational polymorphism analyses (SSCP) (Orita et al., 1989, Proc. Natl. Acad. Sci. USA 86:2766-2770), and PCR analyses (U.S. Patent Nos. 4,683,202; 4,683,195; 4,800,159; and 4,965,188; PCR Strategies, 1995 Innis et al. (eds.), Academic Press, Inc.).

Such diagnostic methods for the detection of a gene-specific mutation can involve, for example, contacting and incubating the viral nucleic acids with one or more labeled nucleic acid reagents including recombinant DNA molecules, cloned genes or degenerate samples thereof, under conditions favorable for the specific annealing of these reagents to their complementary sequences. Preferably, the lengths of these nucleic acid reagents are at least 15 to 30 nucleotides. After incubation, all non-annealed nucleic acids are removed from the nucleic acid molecule hybrid. The presence of nucleic acids which have hybridized, if any such molecules exist, is then detected. Using such a detection scheme, the nucleic acid from the virus can be immobilized, for example, to a solid support such as a membrane, or a plastic surface such as that on a microtiter plate or polystyrene beads. In this case, after incubation, non-annealed, labeled nucleic acid reagents of the type described above are easily removed. Detection of the remaining, annealed, labeled nucleic acid reagents is accomplished using standard techniques well-known to those in the art. The gene sequences to which the nucleic acid reagents have annealed can be compared to the annealing pattern expected from a normal gene sequence in order to determine whether a gene mutation is present.

Alternative diagnostic methods for the detection of gene specific nucleic acid molecules may involve their amplification, *e*.*g*., by PCR (U.S. Patent Nos. 4,683,202; 4,683,195; 4,800,159; and 4,965,188; PCR Strategies, 1995 Innis et al. (eds.), Academic Press, Inc.), followed by the detection of the amplified molecules using techniques well known to those of skill in the art. The resulting amplified sequences can be compared to those which would be expected if the nucleic acid being amplified contained only normal copies of the respective gene in order to determine whether a gene mutation exists.

Additionally, the nucleic acid can be sequenced by any sequencing method known in the art. For example, the viral DNA can be sequenced by the dideoxy method of Sanger et al., 1977, Proc. Natl. Acad. Sci. USA 74:5463, as further described by Messing et al., 1981, Nuc. Acids Res. 9:309, or by the method of Maxam et al., 1980, Methods in Enzymology 65:499. See also the techniques described in Sambrook et al., 2001, Molecular Cloning: A Laboratory Manual, Cold Spring Harbor Laboratory, 3rd ed., NY; and Ausubel et al., 1988 & updates, Current Protocols in Molecular Biology, John Wiley & Sons, NY.

The methods of the instant invention are applicable for determining resistance of an individual viral variant or for determining resistance of a variant population, which may be genotyped simultaneously. For example, for a given sequence, such as PR, sequencing a variant population together provides a genotype that can be used for identification of pertinent PR mutations.

Within the past decade, several technologies have been developed making it possible to identify large numbers (*e*.*g*., hundreds to hundreds of thousands) of nucleic acid sequences in a sample at any one time. *See, e*.*g*., Kozal et al., 1996, Nat. Med. 2(7):753-759; Lockhart et al., 1996, Nature Biotechnology 14:1675-1680; Blanchard et al., 1996, Nature Biotechnology 14, 1649; U.S. Patent No. 5,571,639 issued November 5, 1996. For example, a set oligonucleotide probes of predetermined sequences complimentary to various genotypes of a HIV protease can be attached to specific locations on a solid phase (an array), and the presence or absence of the various sequences in a unknown HIV nucleic acid sequence are determined by the hybridization patterns of the unknown HIV nucleic acid to the probes on the solid-phase. Typically, computer-aided techniques are used to assist in the gathering, processing, and evaluation of the large amount of information garnered in using array-based technology. *See, e.g.,* U.S. Patent No. 6,546,340 issued April 8, 2003. Probe arrays including those made to custom specifications, along with reagents and computer analysis software are all commercially available (*e*.*g*., Affymetrix, Inc., Santa Clara CA).

Identification of a mutation in an HIV-1 protease may be determined by amino acid analysis of the protease. Identification of a mutation in an HIV protease may be determined, for example, by the use of antibodies specifically recognizing particular amino residues at certain positions in HIV protease. Such antibodies can be used in ELISA assays or immunoprecipitation studies to assess the presence of mutant amino acids in the protease. Alternately, the HIV protease may be sequenced directly using techniques conventional in the art. For example, the protease may be digested with different peptidases and the amino acid compositions of the peptide digests determined using mass spectrometry. The resulting peptide sequences can then be assembled into the sequence of the entire HIV protease.

### 5.5 Measuring Phenotypic Susceptibility of a Mutant Virus

Any method known in the art can be used to determine the phenotypic susceptibility of a mutant virus or population of viruses to an anti-viral therapy. In some embodiments a phenotypic analysis is performed, *i*.*e*., the susceptibility of the virus to a given anti-viral agent can be assayed with respect to the susceptibility of a reference virus without the mutations. This is a direct, quantitative measure of drug susceptibility and can be performed by any method known in the art to determine the susceptibility of a virus to an anti-viral agent. An example of a method includes, but is not limited to, determining the fold change in IC₅₀ values with respect to a reference virus. Phenotypic testing measures the ability of a specific viral strain to grow in vitro in the presence of a drug inhibitor. A virus is less susceptible to a particular drug when more of the drug is required to inhibit viral activity, versus the amount of drug required to inhibit the reference virus.

In one embodiment, phenotypic analysis is performed and used to calculate the IC₅₀ or IC₉₀ of a drug for a viral strain. The results of the analysis can also be presented as fold-change in IC₅₀ or IC₉₀ for each viral strain as compared with a drug-susceptible control strain or a prior viral strain from the same patient. Because the virus is directly exposed to each of the available anti-viral medications, results can be directly linked to treatment response. For example, if the patient virus shows resistance to a particular drug, that drug is avoided or omitted from the patient's treatment regimen, allowing the physician to design a treatment plan that is more likely to be effective for a longer period of time.

In another embodiment, the phenotypic analysis is performed using recombinant virus assays ("RVAs"). RVAs use virus stocks generated by homologous recombination between viral vectors and viral gene sequences, amplified from the patient virus. In some embodiments, the viral vector is a HIV vector and the viral gene sequences can be protease and reverse transcriptase sequences.

In one embodiment, the phenotypic analysis is performed using PHENOSENSE^{™} (ViroLogic Inc., South San Francisco, CA). *See* Petropoulos et al., 2000, Antimicrob. Agents Chemother. 44:920-928*;* U.S. Patent Nos. 5,837,464 and 6,242,187. PHENOSENSE^{™} is a phenotypic assay that achieves the benefits of phenotypic testing and overcomes the drawbacks of previous assays. Because the assay has been automated, PHENOSENSE^{™} offers higher throughput under controlled conditions. The result is an assay that accurately defines the susceptibility profile of a patient's HIV isolates to all currently available antiretroviral drugs, and delivers results directly to the physician within about 10 to about 15 days of sample receipt. PHENOSENSE^{™} is accurate and can obtain results with only one round of viral replication, thereby avoiding selection of subpopulations of virus. The results are quantitative, measuring varying degrees of drug susceptibility, and sensitive - the test can be performed on blood specimens with a viral load of about 500 copies/mL and can detect minority populations of some drug-resistant virus at concentrations of 10% or less of total viral population. Furthermore, the results are reproducible and can vary by less than about 1.4-2.5 fold, depending on the drug, in about 95% of the assays performed.

PHENOSENSE^{™} can be used with nucleic acids from amplified viral gene sequences. The sample containing the virus may be a sample from a human or an animal infected with the virus or a sample from a culture of viral cells. In one embodiment, the viral sample comprises a genetically modified laboratory strain.

A resistance test vector ("RTV") can then be constructed by incorporating the amplified viral gene sequences into a replication defective viral vector by using any method known in the art of incorporating gene sequences into a vector. In one embodiment, restrictions enzymes and conventional cloning methods are used. *See* Maniatis et al., 1989, Molecular Cloning: A Laboratory Manual, Cold Spring Harbor Laboratory, NY and Ausubel et al., 1989, Current Protocols in Molecular Biology, Greene Publishing Associates and Wiley Interscience, NY. In some embodiments, *Apa*I and *Pin*AI restriction enzymes are used. Preferably, the replication defective viral vector is the indicator gene viral vector ("IGVV"). In some embodiments, the viral vector contains a means for detecting replication of the RTV. Preferably, the viral vector contains a luciferase expression cassette.

The assay can be performed by first co-transfecting host cells with RTV DNA and a plasmid that expresses the envelope proteins of another retrovirus, for example, amphotropic murine leukemia virus (MLV). Following transfection, virus particles can be harvested and used to infect fresh target cells. The completion of a single round of viral replication can be detected by the means for detecting replication contained in the vector. In one embodiment, the completion of a single round of viral replication results in the production of luciferase. Serial concentrations of anti-viral agents can be added at either the transfection step or the infection step.

Susceptibility to the anti-viral agent can be measured by comparing the replication of the vector in the presence and absence of the anti-viral agent. For example, susceptibility to the anti-viral agent can be measured by comparing the luciferase activity in the presence and absence of the anti-viral agent. Susceptible viruses would produce low levels of luciferase activity in the presence of anti-viral agents, whereas viruses with reduced susceptibility would produce higher levels of luciferase activity.

In one embodiment, PHENOSENSE^{™} is used in evaluating the phenotypic susceptibility of HIV-1 to anti-viral drugs. Preferably, the anti-viral drug is a protease inhibitor. More preferably, it is tipranavir. In some embodiments, the reference viral strain is HIV strain NL4-3 or HXB-2.

In one embodiment, viral nucleic acid, for example, HIV-1 RNA can be extracted from plasma samples, and a fragment of, or entire viral genes could be amplified by methods such as, but not limited to PCR. *See, e.g.,* Hertogs et al., 1998, Antimicrob Agents Chemother 42(2):269-76. In one example, a 2.2-kb fragment containing the entire HIV-1 PR- and RT-coding sequence can be amplified by nested reverse transcription-PCR. The pool of amplified nucleic acid, for example, the PR-RT-coding sequences, can then be cotransfected into a host cell such as CD4+ T lymphocytes (MT4) with the pGEMT3ΔPRT plasmid from which most of the PR (codons 10 to 99) and RT (codons 1 to 482) sequences are deleted. Homologous recombination leads to the generation of chimeric viruses containing viral coding sequences, such as the PR- and RT-coding sequences derived from HIV-1 RNA in plasma. The susceptibilities of the chimeric viruses to all currently available anti-viral agents targeting the products of the transfected genes (proRT and/or PR inhibitors, for example), can be determined by any cell viability assay known in the art. For example, an MT4 cell-3-(4,5-dimethylthiazol-2-yl) -2,5-diphenyltetrazolium bromide-based cell viability assay can be used in an automated system that allows high sample throughput. The profile of resistance to all the anti-viral agents, such as the RT and PR inhibitors can be displayed graphically in a single PR-RT-Antivirogram.
Other assays for evaluating the phenotypic susceptibility of a virus to anti-viral drugs known to one of skill in the art can be used. *See, e.g.,* Shi and Mellors, 1997, Antimicrob Agents Chemother. 41(12):2781-85; Gervaix et al., 1997, Proc Natl Acad Sci U. S. A. 94(9):4653-8,

In another embodiment, the susceptibility of a virus to treatment with an anti-viral treatment is determined by assaying the activity of the target of the anti-viral treatment in the presence of the anti-viral treatment. In one embodiment, the virus is HIV, the anti-viral treatment is a protease inhibitor, and the target of the anti-viral treatment is the HIV protease. *See, e.g.,* U. S. Patent Nos. 5,436,131 and 6,103,462.

### 5.6 Correlating Phenotypic and Genotypic Resistance to Protease Inhibitors

Datasets of viral variants with identified phenotypes can be used to correlate phenotypic and genotypic resistance to protease inhibitors.

A phenotypic analysis can be performed and used to calculate the IC₅₀ or IC₉₀ of a drug for a virus variant. The results of the analysis can also be presented as fold-change in IC₅₀ or IC₉₀ for each variant as compared with a drug-susceptible reference virus or a viral sample taken from the same subject prior to a drug therapy.

Any method known in the art, without limitation, can be used to determine the phenotypic susceptibility or resistance of a mutant virus or population of viruses to an anti-viral therapy. Examples of determining phenotypes may found, for example, in U.S. Patent Nos. 6,653,081, 6,489,098, 6,351,690,6,242,187, and 5,837,464. For example, a phenotypic assay can be performed using the PHENOSENSE^{™} phenotype HIV assay (ViroLogic Inc., South San Francisco, CA), as discussed above. *See* Petropoulos et al., 2000, Antimicrob. Agents Chemother. 44:920-928.

Any method known in the art can be used to determine whether a mutation is correlated with an increase in resistance of a virus to a protease inhibitor. Typically, P values are used to determine the statistical significance of the correlation, such that the smaller the P value, the more significant the measurement. Preferably the P values will be less than 0.05 (or 5%). More preferably, P values will be less than 0.01. P values can be calculated by any means known to one of skill in the art. For the purposes of correlating an increase in resistance of an HIV-1 to a mutation, P values can be calculated using Fisher's Exact Test. *See,* e.g., David Freedman, Robert Pisani & Roger Purves, 1980, STATISTICS, W. W. Norton, New York. P values may be calculated using Student's paired and/or unpaired t-test and the non-parametric Kruskal-Wallis test (Statview 5.0 software, SAS, Cary, NC).

Mutations associated with altered susceptibility to a drug in the HIV-1 protease gene are generally classified into two groups. A first group typically includes those mutations either selected first in the presence of the drug or are otherwise shown to have an effect on drug binding to the protease or an effect on viral activity and replication. A second group of mutations may include mutations that appear later than primary mutations and by themselves do not have a significant effect on resistance phenotype. This second group of mutations are frequently thought to improve replicative fitness caused by mutations of the first group.

Biological samples may include any sample that can contain an HIV, preferably an HIV-1. Biological samples from an HIV-infected subject include, for example and without limitation, blood, blood plasma, serum, urine, saliva, tissue swab and the like.

### 5.7 Constructing an Algorithm

In one aspect, the present invention provides a method of constructing an algorithm that correlates genotypic data about a virus with phenotypic data about the virus. In one embodiment, the phenotypic data relate to the susceptibility of the virus to an anti-viral treatment. In some embodiments, the anti-viral treatment is an anti-viral compound. In some embodiments, the anti-viral compound is a protease inhibitor. In some embodiments, the protease inhibitor is tipranavir.

In one embodiment, the method of constructing the algorithm comprises creating a rule or rules that correlate genotypic data about a set of viruses with phenotypic data about the set of viruses.

In one embodiment, a data set comprising genotypic and phenotypic data about each virus in a set of viruses is assembled. Any method known in the art can be used to collect genotypic data about a virus. Examples of methods of collecting such data are provided above. Any method known in the art can be used for collecting phenotypic data about a virus. Examples of such methods are also provided above. In some embodiments, the data set comprises one or more RAMs as described above. In one embodiment, each genotypic datum is the sequence of all or part of a viral protein of a virus in the set of viruses. In some embodiments, each genotypic datum in the data set is a single amino acid change in a protein encoded by the virus, relative to a reference protein in the reference virus. In some embodiments, the genotype comprises two, three, four, five, six, seven, eight, nine or more amino acid changes in the viral protein. In some embodiments, the virus is HIV, and the protein is HIV protease. In one embodiment, the virus is HIV-1. In another embodiment, the reference protein is the protease from NL4-3 HIV.

In one embodiment, each phenotypic datum in the data set is the susceptibility to an anti-viral treatment of a virus in the set of viruses. In one embodiment, the anti-viral treatment is with an anti-viral compound. In some embodiments, the anti-viral compound is a protease inhibitor. In some embodiments, the protease inhibitor is tipranavir. In one embodiment, the susceptibility is measured as a change in the susceptibility of the virus relative to a reference virus. In another embodiment, the susceptibility is measured as a change in the IC₅₀ of the virus relative to a reference virus. In another embodiment, the change in IC₅₀ is represented as the fold-change in IC₅₀. In one embodiment the virus is HIV. In another embodiment, the virus is HIV-1. In another embodiment, the reference HIV is NL4-3 HIV.

The genotypic and phenotypic data in the data set can be represented or organized in any way known in the art. In one embodiment, the data are displayed in the form of a graph, for example, as shown in Figures 2-3. In this type of representation, the y-axis represents the fold change in IC₅₀ of a virus in the data set relative to a reference virus. Each point on the graph corresponds to one virus in the data set. The x-axis represents the number of mutations that a virus in the data set has. The position of the point indicates both the number of mutations and the fold change in anti-viral therapy treatment that the virus has, both measured relative to a reference strain. In another embodiment, the genotypic and phenotypic data in the data set are displayed in the form of a chart.

In one aspect, an algorithm is formulated that correlates the genotypic data with the phenotypic data in the data set. In one embodiment, a phenotypic cutoff point is defined. In another embodiment, the phenotype is susceptibility to an anti-viral treatment. In another embodiment, the phenotype is change in sensitivity to an anti-viral treatment relative to a reference virus, and the cutoff point is the value above which a virus or population of viruses is defined as phenotypically resistant or partially resistant ("PT-R") to the anti-viral therapy and below which a virus or population of viruses is defined as phenotypically sensitive ("PT-S") to the anti-viral therapy. In certain embodiments, the PT-R virus or population of viruses exhibits reduced susceptibility to the anti-viral treatment. In other embodiments, the cutoff point is 2-fold, 3-fold, 4-fold, 5-fold, 10-fold, 15-fold, 20-fold, 30-fold, 40-fold, 50-fold or 100-fold greater than the IC₅₀ of a reference virus. In another embodiment, the phenotypic cutoff point is the upper or lower cutoff value as defined above. In another embodiment, the virus is HIV and the anti-viral therapy is treatment with a protease inhibitor. In another embodiment, the protease inhibitor is tipranavir.

In another embodiment, the upper or lower clinical cutoff point is used to define a genotypic cutoff point. In one embodiment, the number of mutations in a virus of the data set are correlated with the phenotypic susceptibility of the virus. This can be done, for example, using a graph similar to the one in Figure 2, as discussed above. A genotypic cutoff point can be selected such that most viruses having more than that number of mutations in the data set are phenotypically resistant or partially resistant ("PT-R"), and most viruses having fewer than that number of mutations are phenotypically sensitive ("PT-S"). By definition, a virus in the data set with number of mutations equal to, or more than the genotypic cutoff is genotypically resistant ("GT-R") to the anti-viral treatment, and a virus in the data set with fewer than the genotypic cutoff is genotypically sensitive ("GT-S") to the anti-viral treatment. Thus, in one embodiment, a genotypic cutoff point is selected that produces the greatest percentage of viruses in the data set that are either phenotypically resistant and genotypically resistant ("PT-R, GT-R"), or phenotypically sensitive and genotypically sensitive ("PT-S, GT-S").

While this simple algorithm can provide a useful approximation of the relationship between the genotypic and phenotypic data in the data set, in most cases there will be a significant number of strains that are genotypically sensitive but phenotypically resistant or partially resistant ("GT-S, PT-R"), or genotypically resistant but phenotypically sensitive ("GT-R, PT-S"), as shown in Figures 2-4. These discordant results are a measure of the inaccuracy of the algorithm. Thus, in some embodiments, the algorithm is further modified to reduce the percentage of discordant results in the data set. In one embodiment, this is done by removing from the data set each data point that corresponds to a virus population comprising a mixture of mutations including the wild-type, at a single position considered by the algorithm tested. *See e.g*., U.S. Pat. Pub. No. 2004/0224307. This has the effect of reducing the number of PT-S, GT-R results, thus lowering the overall percentage of discordant results and so improves the fit of the algorithm to a data set.

In another embodiment, the percentage of discordant results is reduced by assigning differential weight values to one or more mutations observed in the data set. An algorithm that does not include this step assumes that each mutation in the data set contributes equally to the overall resistance of a virus or population of viruses to an anti-viral therapy. In many cases this will not be true. For example, there may be a mutation in a data set that is almost always correlated with phenotypic resistance to an anti-viral treatment. That is, almost every virus that has the mutation is phenotypically resistant to the anti-viral treatment, even those strains having only one or two total mutations. In one embodiment, such mutations are "weighted," e.g., assigned an increased mutation score. A mutation can be assigned a weight of, for example, two, three, four, five, six, seven, eight or more. For example, a mutation assigned a weight of 2 can be counted as two mutations in a virus. Fractional weighting values can also be assigned. In certain embodiments, a value between zero and one can be assigned when a mutation is weakly associated with reduced susceptibility to the anti-viral treatment. In another embodiment, values of less than zero, can be assigned, wherein a mutation is associated with an increased sensitivity of the virus to the anti-viral treatment.

One of skill in the art will appreciate that there is a tradeoff involved in assigning an increased weight to certain mutations. As the weight of the mutation is increased, the number of GT-R, PT-S discordant results may increase. Thus, assigning a weight to a mutation that is too great may increase the overall discordance of the algorithm. Accordingly, in one embodiment, a weight is assigned to a mutation that balances the reduction in GT-S, PT-R results with the increase in GT-R, PT-S results.

In another embodiment, the interaction of different mutations in the data set with each other is also factored into the algorithm. For example, it might be found that two or more mutations behave synergistically, *i.e*., that the coincidence of the mutations in a virus contributes more significantly to the resistance of the virus than would be predicted based on the effect of each mutation independent of the other. Alternatively, it might be found that the coincidence of two or more mutations in a virus contributes less significantly to the resistance of the virus than would be expected from the contributions made to resistance by each mutation when it occurs independently. Also, two or more mutations may be found to occur more frequently together than as independent mutations. Thus, in one embodiment, mutations occurring together are weighted together. For example, only one of the mutations is assigned a weight of 1 or greater, and the other mutation or mutations are assigned a weight of zero, in order to avoid an increase in the number of GT-R, PT-S discordant results.

In another aspect, the phenotypic cutoff point can be used to define a genotypic cutoff point by correlating the number as well as the class of mutations in a virus of the data set with the phenotypic susceptibility of the virus. Examples of classes of mutations include, but are not limited to, primary amino acid mutations, secondary amino acid mutations, mutations in which the net charge on the polypeptide is conserved and mutations that do not alter the polarity, hydrophobicity or hydrophilicity of the amino acid at a particular position. Other classes of mutations that are within the scope of the invention would be evident to one of skill in the art, based on the teachings herein.

In one embodiment, an algorithm is constructed that factors in the requirement for one or more classes of mutations. In another embodiment, the algorithm factors in the requirement for a minimum number of one or more classes of mutations. In another embodiment, the algorithm factors in the requirement for a minimum number of primary or secondary mutations. In another embodiment, the requirement for a primary or a secondary mutation in combination with other mutations is also factored into the algorithm. For example, it might be found that a virus with a particular combination of mutations is resistant to an anti-viral treatment, whereas a virus with any mutation in that combination, alone or with other mutations that are not part of the combination, is not resistant to the anti-viral treatment.

By using, for example, the methods discussed above, the algorithm can be designed to achieve any desired result. In one embodiment, the algorithm is designed to maximize the overall concordance (the sum of the percentages of the PT-R, GT-R and the PT-S, GT-S groups, or 100 - (percentage of the PT-S, GT-R + PT-R, GT-S groups). In some embodiments, the overall concordance is greater than 75%, 80%, 85%, 90% or 95%. In one embodiment, the algorithm is designed to minimize the percentage of PT-R, GT-S results. In another embodiment, the algorithm is designed to minimize the percentage of PT-S, GT-R results. In another embodiment, the algorithm is designed to maximize the percentage of PT-S, GT-S results. In another embodiment, the algorithm is designed to maximize the percentage of PT-R, GT-R results.

At any point during the construction of the algorithm, or after it is constructed, it can be further tested on a second data set. In one embodiment, the second data set consists of viruses that are not included in the data set, *i.e*., the second data set is a naïve data set. In another embodiment, the second data set contains one or more viruses that were in the data set and one or more viruses that were not in the data set. Use of the algorithm on a second data set, particularly a naïve data set, allows the predictive capability of the algorithm to be assessed. Thus, in one embodiment, the accuracy of an algorithm is assessed using a second data set, and the rules of the algorithm are modified as described above to improve its accuracy. In another embodiment, an iterative approach is used to create the algorithm, whereby an algorithm is tested and then modified repeatedly until a desired level of accuracy is achieved.

### 5.8 Using an Algorithm to Predict the Susceptibility of a Virus

In another aspect, the present invention also provides a method for using an algorithm of the invention to predict the phenotypic susceptibility of a virus or a derivative of a virus to an anti-viral treatment based on the genotype of the virus. In one embodiment, the method comprises detecting, in the virus or derivative of the virus, the presence or absence of one or more mutations associated with altered susceptibility to tipranavir, applying the rules of the algorithm to the detected mutations associated with altered susceptibility to tipranavir, wherein a virus that satisfies the rules of the algorithm is genotypically resistant or partially resistant to the anti-viral treatment, and a virus that does not satisfy the rules of the algorithm is genotypically sensitive to the anti-viral treatment. In certain embodiments, a virus that is partially resistant or resistant to the treatment exhibits reduced susceptibility to the treatment.

In another embodiment, the method comprises detecting, in the virus or derivative of the virus, the presence or absence of one or more mutations associated with altered susceptibility to tipranavir, applying the rules of the algorithm to the detected mutations, wherein a score equal to, or greater than the genotypic cutoff score indicates that the virus is genotypically resistant or partially resistant to the anti-viral treatment, and a score less than the genotypic cutoff score indicates that the virus is genotypically sensitive to the anti-viral treatment. In certain embodiments, a virus that is partially resistant or resistant to the treatment exhibits reduced susceptibility to the treatment.

In yet another embodiment, the method comprises detecting, in the virus or derivative of the virus, the presence or absence of one or more mutations associated with altered susceptibility to tipranavir, applying the rules of the algorithm to the detected mutations, wherein a score less zero indicates that the virus exhibits increased susceptibility to the anti-viral treatment.

The algorithm of this invention can be used for any viral disease where anti-viral drug susceptibility is a concern, as discussed above. In certain embodiments the assay of the invention can be used to determine the susceptibility of a retrovirus to an anti-viral drug. In one embodiment, the retrovirus is HIV. Preferably, the virus is HIV-1.

The anti-viral agent of the invention could be any treatment effective against a virus. It is useful to the practice of this invention, for example, to understand the structure, life cycle and genetic elements of the viruses which can be tested in the drug susceptibility test of this invention. These would be known to one of ordinary skill in the art and provide, for example, key enzymes and other molecules at which the anti-viral agent can be targeted. Examples of anti-viral agents include, but are not limited to, nucleoside reverse transcriptase inhibitors such as AZT, ddI, ddC, d4T, 3TC, abacavir, nucleotide reverse transcriptase inhibitors such as tenofovir, non-nucleoside reverse transcriptase inhibitors such as nevirapine, efavirenz, delavirdine, fusion inhibitors such as T-20 and T-1249 and protease inhibitors such as saquinavir, ritonavir, indinavir, nelfinavir, amprenavir, atazanavir, lopinavir and tipranavir.

In some embodiments of the invention, the anti-viral agents are directed at retroviruses. In certain embodiments, the anti-viral agents are protease inhibitors such as saquinavir, ritonavir, indinavir, nelfinavir, amprenavir, atazanavir, lopinavir, and tipranavir. In one embodiment, the anti-viral agent is tipranavir.

Any mutation, without limitation, associated with reduced susceptibility to treatment with an anti-viral agent known to those in the art can be used in the algorithms of the invention. Additional mutations useful in the algorithms of the invention can be determined by methods described above. For example, Tables 2 and 3 provide a list of mutations associated with reduced susceptibility to tipranavir.

### 5.9 Using an Algorithm to Predict the Effectiveness of Anti-Viral Treatment for an Individual

In another aspect, the present invention also provides a method for using an algorithm of the invention to predict the effectiveness of an anti-viral treatment for an individual infected with a virus based on the genotype of the virus. In certain embodiments, the method comprises detecting, in the virus or derivative of the virus, the presence or absence of one or more mutations associated with altered susceptibility to an antiviral drug, applying the rules of the algorithm to the detected mutations, wherein a virus that satisfies the rules of the algorithm exhibits reduced susceptibility to the anti-viral treatment, and a virus that does not satisfy the rules of the algorithm is genotypically sensitive to the anti-viral treatment, thereby identifying the effectiveness of the anti-viral treatment. In certain embodiments, the virus exhibiting reduced susceptibility to the treatment is partially resistant. In certain embodiments, the virus exhibiting reduced susceptibility to the treatment is resistant. In certain embodiments, the method comprises detecting, in the virus or a derivative of the virus, the presence or absence of one or more mutations, applying the rules of the algorithm to the detected mutations, wherein a score equal to, or greater than the genotypic cutoff score indicates that the virus exhibits reduced susceptibility to the anti-viral treatment, and a score less than the genotypic cutoff score indicates that the virus is genotypically sensitive to the anti-viral treatment. In certain embodiments, the virus exhibiting reduced susceptibility to the treatment is partially resistant.

As described above, the algorithm of the invention can be used for any viral disease where anti-viral drug susceptibility is a concern and the anti-viral agent of the invention could be any treatment effective against a virus. In certain embodiments the assay of the invention is used to determine the susceptibility of a retrovirus to an anti-viral drug. In a preferred embodiment, the retrovirus is HIV. Preferably, the virus is HIV-1. In some embodiments of the invention, the anti-viral agents are directed at retroviruses. In certain embodiments, the anti-viral agents are protease inhibitors such as saquinavir, ritonavir, indinavir, nelfinavir, amprenavir, atazanavir and lopinavir. In one embodiment, the anti-viral agent is tipranavir.

As described above, mutations associated with reduced susceptibility to treatment with an anti-viral agent may be obtained from the art or determined by methods described in Sections 5.3 - 5.8.

In some embodiments, the present invention provides a method for monitoring the effectiveness of an anti-viral treatment in an individual infected with a virus and undergoing or having undergone prior treatment with the same or different anti-viral treatment, comprising, detecting, in a sample of said individual, the presence or absence of an amino acid residue associated with reduced susceptibility to treatment with the anti-viral treatment, wherein the presence of the residue correlates with a reduced susceptibility to treatment with the anti-viral treatment.

### 5.10 Correlating Susceptibility to one Anti-Viral Treatment with Susceptibility to Another Anti-Viral Treatment

In another aspect, the present invention provides a method for using an algorithm of the invention to predict the effectiveness of an anti-viral treatment against a virus based on the genotypic susceptibility of the virus to a different anti-viral treatment. In certain embodiments, the method comprises detecting, in a virus or a derivative of a virus, the presence or absence of one or more mutations correlated with altered susceptibility to an anti-viral treatment and applying the rules of an algorithm of the invention to the detected mutations, wherein a virus that satisfies the rules of the algorithm is genotypically resistant or partially resistant to the anti-viral treatment, and a virus that does not satisfy the rules of the algorithm is genotypically sensitive to the anti-viral treatment. In certain embodiments, the method comprises detecting, in the virus or a derivative of the virus, the presence or absence of one or more mutations correlated with altered susceptibility to an anti-viral treatment and applying the rules of the algorithm to the detected mutations, wherein a score equal to, or greater than the genotypic cutoff score indicates that the virus is genotypically resistant or partially resistant to a different anti-viral treatment, and a score less than the genotypic cutoff score indicates that the virus is genotypically sensitive to a different anti-viral treatment. In another embodiment, the two anti-viral treatments affect the same viral protein. In another embodiment, the two anti-viral treatments are both protease inhibitors. Examples of protease inhibitors include, but are not limited to, saquinavir, ritonavir, indinavir, nelfinavir, amprenavir, atazanavir, lopinavir and tipranavir. In yet another embodiment, one of the two anti-viral treatments is tipranavir. In still another embodiment, a mutation correlated with resistance to one protease inhibitor is also correlated with resistance to another protease inhibitor.

### 5.11 Computer Implemented Methods

In one aspect, the present invention provides a computer implemented method of identifying an HIV-1 as being less susceptible to tipranavir therapy in a subject infected with the HIV-1. Typically, data representing the HIV-1 genotype is received as input by a computer system. For example, data can be entered by a keyboard. As another example, data can be received electronically from a device used for the purpose of genotyping nucleic acids. Typically, genotyping of HIV nucleic acids is resolved by electrophoretic methods using dye termination chemistry reactions, although other options are possible including hybridization patterns of an HIV nucleic acid to an oligonucleotide array. Thus, the data received as input may represent electrophoretic migrations or hybridization patterns which can be converted into a representation of a genotype.

Embodiments of the computer implemented method comprise performing comparison of the genotype of the HIV-1 to a database representing pertinent mutations associated with altered susceptibility to protease inhibitors. In preferred embodiments, the database comprises representations of mutant protease codons L10I/V, I13V, K20M/V/R, L24I, D30N, V32I, L33F, E35G, M36I, K43T, M46I/L, I47V, I50L/V, I54A/L/M/V, Q58E, H69K, A71L, G73T, T74P, L76V, V82I/L/T, N83D, I84V, L89V, and/or L90M. Performing a comparison between the genotype of the HIV-1 and the database can be performed in any sequential order, without limitation, and does not depend on considerations such as amino acid position in the protease or whether a particular position represents a site of a primary or secondary mutation; it is only required that performing a comparison is undertaken in such a way that the recited conditions can be determined.

In one aspect, the present invention provides a computer implemented method of determining that an HIV-1 is likely to be resistant to tipranavir, comprising inputting into a computer system a genotype of HIV-1 protease from the HIV-1 and comparing, thereby the genotype of the HIV-1 protease to a database in the computer system that comprises a correlation between the presence of a mutation at 10, 11, 13, 20, 24, 30, 32, 33, 35, 36,43, 46, 47, 50, 54, 58, 69, 71, 73, 74, 76, 82, 83, 84, 89 and/or 90 and altered susceptibility to tipranavir, calculating a mutation score, wherein if the mutation score is equal to or greater than a cutoff score, the HIV-1 is determined to be resistant to tipranavir. In certain embodiments, the mutation is L10I/V, I13V, K20M/V/R, L24I, D30N, V32I, L33F, E35G, M36I, K43T, M46I/L, I47V, I50L/V, I54A/L/M/V, Q58E, H69K, A71L, G73T, T74P, L76V, V82I/L/T, N83D, I84V, L89V, and/or L90M.

In one embodiment, a computer implemented method comprises determining whether a condition is met that the mutation score is equal to or greater than a cutoff score.

In another aspect, the invention provides a computer implemented method for determining whether a HIV-1 has an increased likelihood of having a reduced susceptibility to treatment with a protease inhibitor, such as tipranavir, comprising inputting into a computer system a genotype of HIV-1 protease from the HIV-1 and comparing the genotype of the HIV-1 protease to a database in the computer system that comprises a correlation between the presence of a mutation at amino acid position 10, 11, 32, 36, 46, 55, 60, 71, 73, 84, 89 and/or 90 of the amino acid sequence of said protease and reduced susceptibility to treatment with the protease inhibitor, thereby determining whether the HIV-1 has an increased likelihood of having reduced susceptibility to the protease inhibitor.

In some embodiments, the mutation associated with reduced susceptibility to treatment with said protease inhibitor is selected from the group consisting of: L1I, V11L, V32I, M46I, K55R, D60E, A71L, G73T, I84V, L89V and L90M.

In another aspect, the invention provides a computer implemented method for determining whether a HIV-1 has an increased likelihood of having increased susceptibility to treatment with a protease inhibitor, such as tipranavir, comprising inputting into a computer system a genotype of HIV-1 protease from the HIV-1 and comparing the genotype of the HIV-1 protease to a database in the computer system that comprises a correlation between the presence of a mutation at amino acid position 10, 24, 30, 50, 54, 76, 82 and/or 88 of the amino acid sequence of said protease and increased susceptibility to treatment with the protease inhibitor, thereby determining whether the HIV-1 has an increased likelihood of having increased susceptibility to the protease inhibitor.

In some embodiments, the mutation associated with increased susceptibility to treatment with said protease inhibitor is selected from the group consisting of: L10F, L24I, D30N, I50L/V, I54L, L76V, V82I and N88D.

The computer implemented methods disclosed herein may be implemented on any computer that is known to those of skill in the art, without limitation. It will be recognized that the implemented methods disclosed herein do not depend on a particular type of computer, memory storage elements, processing speeds, programming languages, compilers, other computer hardware, software or peripherals, and the like.

In certain embodiments, the computer implemented methods comprise displaying a result indicating whether or not the HIV-1 is determined to be less susceptible to tipranavir therapy in a subject infected with the HIV-1. It is generally understood that an output device is used for the display of the results obtained using the computer-implemented methods of the invention. Output devices can be any type of printers, computer screens, disk drives, CD writers, other computers, or memory modules accessible by another computer, and the like, without limitation. Displaying a result can be any display known to those of skill in the art without limitation.

In certain embodiments, the result is displayed on a tangible medium. Typically, results are displayed on computer screens, printouts, CDs, and the like. In certain embodiments, the result is outputted as data on a tangible medium. In certain embodiments, the tangible medium is a paper, *e.g*., a computer printout. In certain embodiments, the tangible medium is a computer-readable memory, *e.g*., a random-access memory, a fixed disk drive, a floppy disk, a compact disk, an iPod™, a flash memory, *etc.*

### 5.12 Other Methods

Those of skill in the art recognize the value of providing the information that can be obtained using the methods disclosed herein. For example, costly yet ineffective antiviral drug treatment regimens can be avoided with the knowledge that an HIV-1 is resistant or partially resistant, exhibiting a reduced susceptibility, to a protease inhibitor, such as tipranavir.

In one aspect, the present invention provides a method that comprises determining whether an HIV-1 is likely to be resistant or partially resistant to a protease inhibitor according to a method of the invention, then providing information disclosing whether an HIV-1 taken from an HIV-1-infected subject is resistant or partially resistant to tipranavir. This information may be provided to the subject or to a health care professional. In certain embodiments, the information further comprises informing the subject or health care professional of the treatment option of treating the subject with tipranavir. In certain embodiments, the information further comprises recommending that the subject or health care professional treat the subject with tipranavir. In certain embodiments, the information further comprises recommending that the subject or health care professional not treat the subject with tipranavir.

In one embodiment, the method comprises obtaining a genotype for nucleic acid encoding protease of the HIV-1. This can be performed, for example, by determining the genotype of the nucleic acid encoding protease from the HIV-1-infected subject and using techniques as described herein, or, for example by receiving genotypic information about the HIV-1's protease from another who genotyped the HIV-1.

In another embodiment, the method comprises identifying the presence or absence of a mutation in the HIV-1 protease that is L10I/V, I13V, K20M/V/R, L24I, D30N, V32I, L33F, E35G, M36I, K43T, M46I/L, I47V, I50L/V, I54A/L/M/V, Q58E, H69K, A71L, G73T, T74P, L76V, V82I/L/T, N83D, I84V, L89V, and/or L90M, and detecting whether the HIV-1 is likely to be resistant or partially resistant to tipranavir.

In one embodiment, the method further comprises preparing a tangible medium indicating whether the HIV-1 is resistant or partially resistant to tipranavir.

In one embodiment, the method further comprises conveying the tangible medium to the subject or a health care provider.

### 5.13 Devices and Systems

In another aspect, the present invention provides a computer system that is configured to perform the computer implemented methods described above. Computers are particular helpful in the performance of the instant methods given the amount of genotype data in combination with rapidity of computers in performing algorithms.

In one embodiment, the computer system comprises a desktop computer running Microsoft WINDOWS^{®} operating system.

In another embodiment, the computer system comprises software written in PERL.

In another aspect, the present invention provides a paper display of the result produced by the methods disclosed herein,

In yet another aspect, the invention provides an article of manufacture that comprises computer-readable instructions for performing the computer-implemented methods discussed above. One embodiment is a CD. Another embodiment is a CD wherein the computer-readable instructions are in PERL.

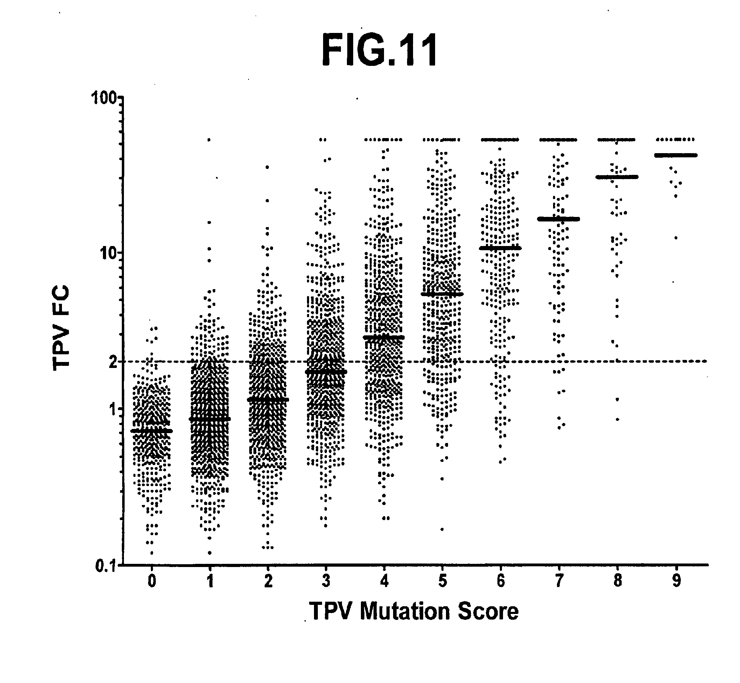

Fig. 11 details an exemplary system that supports the functionality described above. The system is preferably a computer system 10 having:
- a central processing unit 22;
- a main non-volatile storage unit 14, for example, a hard disk drive, for storing software and data, the storage unit 14 controlled by storage controller 12;
- a system memory 36, preferably high speed random-access memory (RAM), for storing system control programs, data, and application programs, comprising programs and data loaded from non-volatile storage unit 14; system memory 36 may also include read-only memory (ROM);
- a user interface 32, comprising one or more input devices (*e.g*., keyboard 28) and a display 26 or other output device;
- a network interface card 20 for connecting to any wired or wireless communication network 34 (*e.g.,* a wide area network such as the Internet);
- an internal bus 30 for interconnecting the aforementioned elements of the system; and
- a power source 24 to power the aforementioned elements.

Operation of computer 10 is controlled primarily by operating system 40, which is executed by central processing unit 22. Operating system 40 can be stored in system memory 36. In addition to operating system 40, in a typical implementation system memory 36 includes:
- file system 42 for controlling access to the various files and data structures used by the present invention;
- a data structure 44 for storing profiles in accordance with the present invention; and
- a data analysis algorithm module 54 for comparing profiles in accordance with the present invention.

As illustrated in Fig. 11, computer 10 comprises software program modules and data structures. Each of the data structures can comprise any form of data storage system including, but not limited to, a flat ASCII or binary file, an Excel spreadsheet, a relational database (SQL), or an on-line analytical processing (OLAP) database (MDX and/or variants thereof). In some specific embodiments, such data structures are each in the form of one or more databases that include hierarchical structure (*e.g*., a star schema). In some embodiments, such data structures are each in the form of databases that do not have explicit hierarchy (*e.g*., dimension tables that are not hierarchically arranged).

In some embodiments, each of the data structures stored or accessible to system 10 are single data structures. In other embodiments, such data structures in fact comprise a plurality of data structures (*e.g*., databases, files, archives) that may or may not all be hosted by the same computer 10. For example, in some embodiments, data structure 44 comprises a plurality of Excel spreadsheets that are stored either on computer 10 and/or on computers that are addressable by computer 10 across wide area network 34. In another example, data structure 44 comprises a database that is either stored on computer 10 or is distributed across one or more computers that are addressable by computer 10 across wide area network 34.

It will be appreciated that many of the modules and data structures illustrated in Figure 22 can be located on one or more remote computers. For example, some embodiments of the present application are web service-type implementations. In such embodiments, a data analysis algorithm module 54 and/or other modules can reside on a client computer that is in communication with computer 10 via network 34. In some embodiments, for example, a data analysis algorithm module 54 can be an interactive web page.

In another aspect, the present invention provides a computer program product comprising one or more computer codes that identify an HIV-1 as being less susceptible to tipranavir treatment in a subject infected with HIV-1 and a computer readable medium that stores the computer codes. In one embodiment, the computer program comprises a computer code that receives input corresponding to the genotype of the HIV-1 nucleic acid encoding HIV-1 protease. The input may represent the nucleotide sequence of the HIV-1 nucleic acid, for example, a list of bases. The input may be converted from a hybridization pattern of the HIV-1 nucleic acid onto an oligonucleotide probe array attached to a solid phase. The input may be converted from an automated sequencer detecting electrophoretic migration.

In another embodiment, the computer program comprises a computer code that performs a first comparison to determine if an amino acid encoded by HIV-1 protease codons 10, 11, 13, 20, 24, 30, 32, 33, 35, 36, 43, 46, 47, 50, 54, 58, 69, 71, 73, 74, 76, 82, 83, 84, 89 and/or 90 matches one or more of mutant amino acids L10I/V, I13V, K20M/V/R, L24I, D30N, V32I, L33F, E35G, M36I, K43T, M46I/L, 147V, I50L/V, I54A/L/M/V, Q58E, H69K, A71L, G73T, T74P, L76V, V82I/L/T, N83D, I84V, L89V, and/or L90M, and a computer code that calculates a mutation score based on the number of mutations present.

In another embodiment, the computer program comprises a computer code that determines whether a condition is met such that the mutation score is equal to or greater than the cutoff score.

In another embodiment, the computer program comprises a computer code conveys a result representing whether or not the HIV-1 is identified as being less susceptible to tipranavir treatment to an output device. An output device may be any known to those of skill in the art, without limitation, such as a printer, a disk drive, a computer screen, another computer, and so forth.

In an aspect, the present invention provides a tangible medium storing the result conveyed to an output device as described above. A tangible medium may be any tangible medium known to those of skill in the art without limitation. A tangible medium may be a CD or DVD. A tangible medium may be a printout. A tangible may be a computer-readable medium as described above.

### 6. EXAMPLES

The following examples are provided to illustrate certain aspects of the present invention and not intended as limiting the subject matter thereof.

### 6.1 Example 1: Analysis of Patient Samples to Identify Mutations associated with reduced susceptibility to tipranavir

This example demonstrates a method of analyzing patient samples so as to identify mutations that are associated either with increased or with decreased susceptibility to protease inhibitors such as tipranavir.

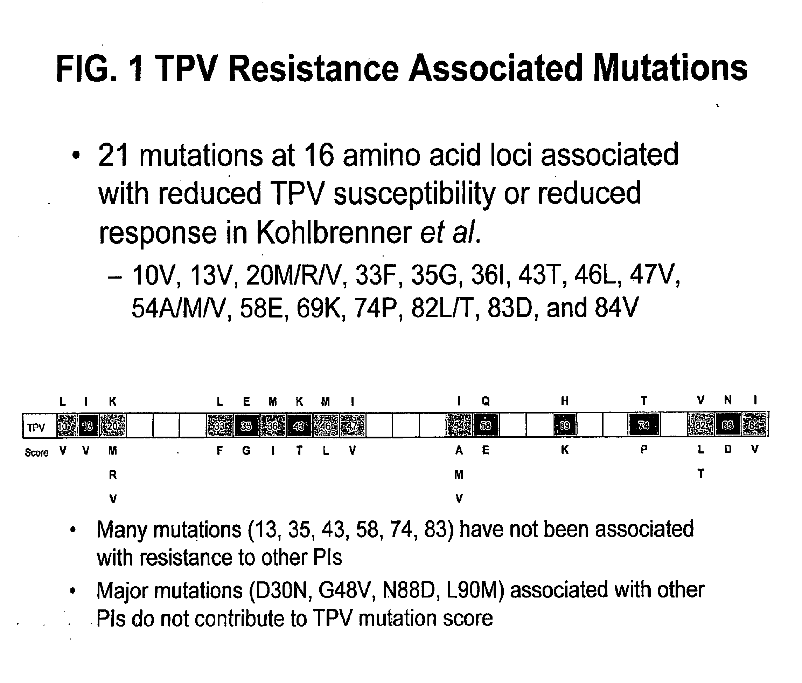

In order to determine the relationship between an HIV-1 strain's protease sequence and its susceptibility to treatment with tipranavir, a dataset consisting of 1411 patient samples was analyzed genotypically as well as phenotypically. The patient samples contained at least one protease inhibitor resistance mutation (L23X, L24X, D30X, V32X, M46X, I47X, G48X, I50X, I54X, V82A, V82F, V82S, V82T, V82C, V82G, V82L, V82M, I84X, N88X, L90X, where X is any non-wild-type amino acid). In addition, the patient sample contained no more than one mixture of amino acid residues at one or more position associated with altered susceptibility to tipranavir used in the Kohlbrenner study (Kohlbrenner *et al*., meeting presentation entitled "Tipranavir Mutation Score Predicts ViroLogic Success in PI-Experienced HIV-Positive Adults," presented at HIV DART Conference (December 2004)). In Kohlbrenner *et al.*, 10V, 13V, 20M/R/V, 33F, 35G, 36I, 43T, 46L, 47V, 54A/M/V, 58E, 69K, 74P, 82L/T, 83D and 84V of HIV-1 protease were identified as mutations associated with altered tipranavir resistance, as shown in Fig. 1. These results were later published as Baxter et al., 2006, J Virol 80:10794-801.

The phenotypic assay was conducted using the PHENOSENSE^{™} (Monogram Biosciences, Inc, South San Francisco, CA) HIV assay (Petropoulos et al., 2000, Antimicrob. Agents Chemother. 44:920-928; U.S. Patent Nos. 5,837,464 and 6,242,187, and International Patent Application No. PCT/US06/05512). Plasma samples were collected from HIV-1-infected patients. IC₅₀ values for tipranavir were obtained for the HIV-1 from the patient sample. This was compared to the IC₅₀ for tipranavir against the NL4-3 (GenBank Accession No. AF324493) reference viral strain. Phenotypic data were expressed as "fold change" (or log fold change) in 50% inhibitory concentration (IC₅₀) of tipranavir. The fold IC₅₀ values were calculated by dividing the IC₅₀ of tipranavir against the HIV-1 from the patient plasma sample by the IC₅₀ for tipranavir against the NL4-3 (GenBank Accession No. AF324493) reference viral strain.

In order to define the genotypic changes correlated with reduced susceptibility to tipranavir, the entire amino acid sequences of HIV-1 proteases in each of the patients' samples were analyzed. Mutations were compared to the protease sequence of the NL4-3 (GenBank Accession No. AF324493) reference strain.

### 6.2 Example 2: Correlation of Tipranavir Susceptibility to Number of Mutations in HIV-1 Protease

This example shows a simple algorithm that correlates the number of equally-weighted mutations in the protease gene of an HIV-1 with its susceptibility to tipranavir, such as described in the Kohlbrenner study, does not predict resistance perfectly well.

A data set of 1411 patient plasma samples was analyzed and mutations associated with reduced susceptibility to tipranavir were identified, as described in Example 1. The genotype data was obtained by sequencing the protease of the HIV-1 present in each patient's sample and determining the sequence changes with respect to the sequence of the NL4-3 (GenBank Accession No. AF324493) HIV.

The phenotypic susceptibility to tipranavir (Log tipranavir fold change) was analyzed as a function of the number of mutations in the protease of the HIV-1 present in a patient's plasma sample. The fold change (FC) for each sample was calculated by dividing the IC₅₀ of tipranavir against the HIV-1 from the patient's plasma sample by the IC₅₀ for tipranavir against the NL4-3 (GenBank Accession No. AF324493) reference viral strain. Samples with offscale tipranavir resistance were assigned a FC value of 100.

Samples were grouped based on a mutation score, which is calculated by adding the number of the mutations associated with tipranavir resistance described in the Kohlbrenner study. The median FC for each group was calculated.

Table 1 shows the relationship between the resistance to tipranavir and the number of the resistance-associated mutations. The mutations used in this analysis were those identified in the Kohlbrenner study As shown in Table 1, among 1411 patient samples in the dataset, 202 samples had a mutation score of zero with a median tipranavir FC of 0.7; 308 samples had a mutation score of one with a median tipranavir FC of 0.8; 241 samples had a mutation score of two with a median tipranavir FC of 1.1; 218 samples had a mutation score of three with a median tipranavir FC of 1.6; 187 samples had a mutation score of four with a median tipranavir FC of 3.0; 119 samples had a mutation score of five with a median tipranavir FC of 5.7; 81 samples had a mutation score of six with a median tipranavir FC of 8.8; 218 samples had a mutation score of seven with a median tipranavir FC of 12; and 22 samples had a mutation score of eight or nine with a median tipranavir FC of 24. The results showed that tipranavir FC of samples increased with increased number of mutations associated with reduced susceptibility to tipranavir.

Fig. 2 and Fig. 3 show the susceptibility to tipranavir (Log tipranavir FC) as a function of the number of mutations associated with reduced susceptibility to tipranavir. Using the mutation score from the Kohlbrenner study, and a phenotypic cutoff of 4-fold, minimum discordance was observed using a mutation score cutoff of four or more mutations at these sixteen positions; therefore, if the number of these mutations were four or more, then the virus was predicted to exhibit reduced susceptibility, to tipranavir treatment.

Fig. 2 shows the results when the mutation score cutoff is five and an HIV-1 was defined as resistant or partially resistant to treatment with tipranavir if its tipranavir FC is greater than or equal to 4. The bottom left quadrant corresponds to those viruses which contain fewer than five mutations in their protease and which are phenotypically and genotypically sensitive (PT-S, GT-S) to tipranavir. 73% of the 1411 samples were found in this quadrant. The top right quadrant corresponds to those viruses which contain five or more mutations and are phenotypically and genotypically resistant or partially resistant (PT-R, GT-R) to tipranavir (tipranavir fold change ≥ 4) and contained 13% of the samples.

However, the other two quadrants correspond to the "exceptions" where a virus was predicted based on genotype (number of mutations) to be sensitive, but was phenotypically (based on tipranavir fold change) resistant or partially resistant (left top, PT-R, GT-S) or where a virus was predicted based on genotype to be resistant or partially resistant, but was phenotypically (based on tipranavir fold change) sensitive (right bottom, PT-S, GT-R).

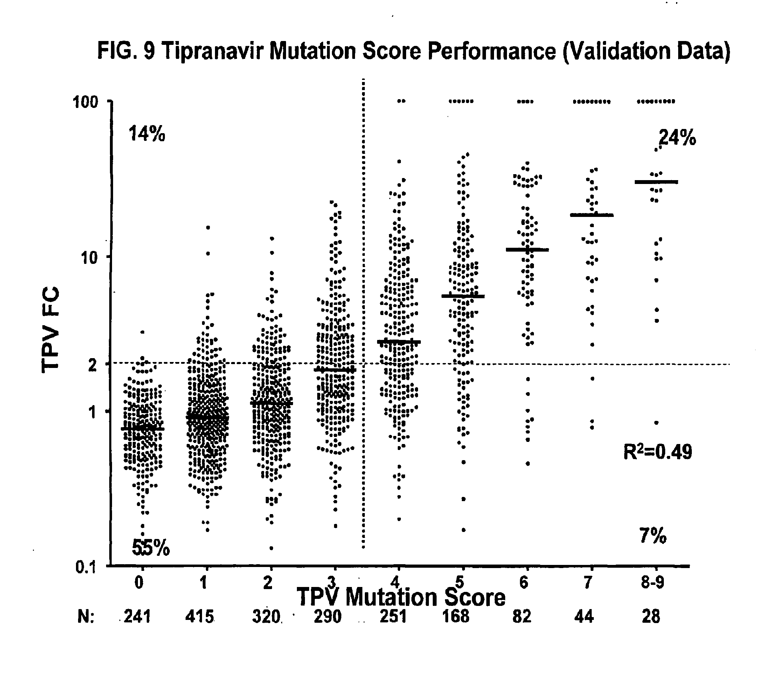

In Fig. 2, 9% of the starting set, with zero to four mutations, contrary to expectations, are found in the top, left (PT-R, GT-S) quadrant and exhibit IC₅₀ values as much as 4- to 100-fold higher than the IC₅₀ for the reference strain (log fold change is 1-2). Conversely, 5% of virus samples that had five, six, seven, eight or nine mutations did not exhibit any reduced susceptibility to tipranavir than the cutoff, and so appear in the bottom, right (PT-S, GT-R) quadrant.

Fig. 3 shows the results when the cutoff mutation score is 4; an HIV-1 was defined as having reduced susceptibility to treatment with tipranavir if its tipranavir FC is greater than or equal to 2. The bottom left quadrant corresponds to those viruses which contain four or fewer mutations in their protease and which are phenotypically and genotypically sensitive (PT-S, GT-S) to tipranavir. 58% of the 1411 samples were found in this quadrant. The top right quadrant corresponds to those viruses which contain four or more mutations and are phenotypically and genotypically having a reduced susceptibility (PT-R, GT-R) to tipranavir (tipranavir fold change ≥ 2) and contained 24% of the samples. 8% of samples, with zero to three mutations, contrary to expectations, are found in the top, left (PT-R, GT-S) quadrant and exhibit IC₅₀ values as much as 2- to 100-fold higher than the IC₅₀ for the reference strain (log fold change is 1-2). Conversely, 10% of virus samples that had four, five, six, seven, eight or nine mutations did not exhibit any reduced susceptibility to tipranavir than the cutoff, and so appear in the bottom, right (PT-S, GT-R) quadrant.

### 6.3 Example 3: Construction of a New Algorithm

This example shows an improved algorithm that correlates the number of mutations in the protease gene of an HIV-1 with its susceptibility to tipranavir.

A data set of 1411 patient plasma samples was analyzed and mutations associated with reduced susceptibility to tipranavir were identified, as described in Example 1 and 2.

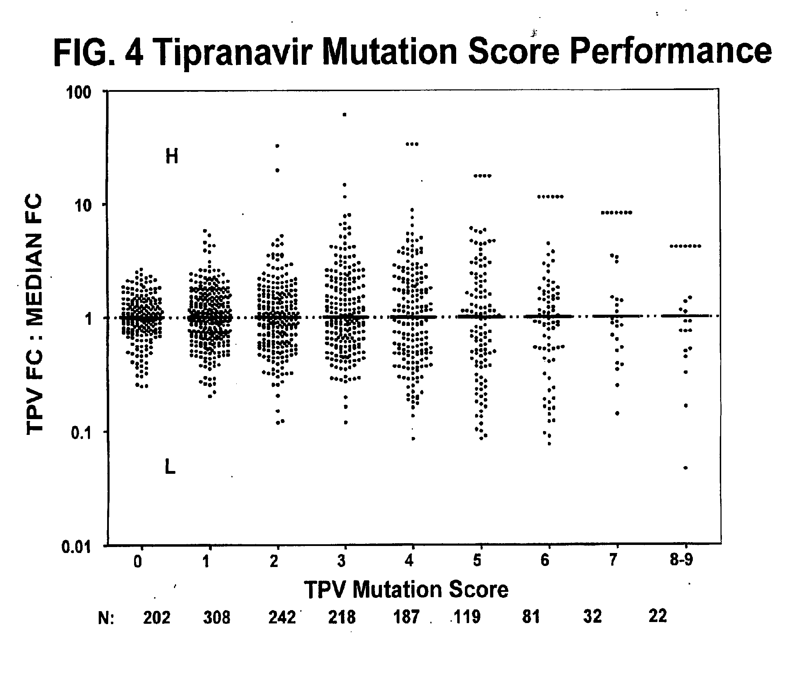

Samples were grouped based on a mutation score, which is calculated by adding the number of the mutations associated with tipranavir resistance described in Kohlbrenner *et al.* The median fold tipranavir FC for samples in each group defined by mutation score was calculated and samples were grouped based on the measured tipranavir FC being higher (H) or lower (C) than the median for each group, as shown in Fig. 4. The odds ratio (OR) for each mutation (% of H samples with the mutation to % of L samples with the mutation was calculated. Fisher's Exact test with Benjamini correction for multiple comparisons was used to determine which protease mutations were associated with H or L tipranavir FC. *See, e.g.,* Benjamini et al., 1995, J. R. Stat. Soc. Ser. B 57:289-300.

Fisher Exact test results as provided in Table 2 show that certain mutations, such as mutations L10I, V11L, V32I, M36L, M46I, I47V, I54A, K55R, D60E, A71L, G73T, V82T, I84V, L89V, and L90M of HIV-1 protease were over-represented in samples with high tipranavir FC (OR>1, adjusted P value <0.05). Conversely, certain mutations, such as L10F or V, I13V, K20R, L24I, D30N, M36I, M46L, I50L or V, I54L, L76V, V82I and N88D were associated with low tipranavir FC (OR<1, adjusted P value <0.05).

Among these mutations identified as associated with altered susceptibility to tipranavir, L10I, V11L, V32I, M36L, M46I, K55R, D60E, A71L, G73T, L89V, and L90M, and L10F, L24I, D30N, I50L or V, I54L, L76V, V82I and N88D were not used and taken into account to calculate mutation score in the Kohlbrenner study. In addition, some mutations, such as I54A, V82T, I47V or I84V were highly over-represented in samples with high tipranavir FC, which may indicate that these mutations contribute more significantly to tipranavir resistance than other mutations. In contrast, some mutations, such as L24I, D30N, I50L/V, I54L, L76V and V82I are or were highly over-represented in samples with low tipranavir FC, which may indicate that these mutations contribute to increased susceptibility to tipranavir.

A new algorithm based on the information from the above results was formulated. In addition to the twenty one mutations at sixteen amino acid position in the Kohlbrenner *et al.* study, sixteen mutations at fifteen amino acid positions that were newly identified as being associated with altered susceptibility to tipranavir were included in the new algorithm. Such mutations include L10I, V11L, L24I, D30N, V32I, M36L, M46I, I50L/V, I54L, A71L, G73T, L76V, V82I, L89V, and L90M of HIV-1 protease. In addition, mutations I47V, I54A, V82T and I84V of HIV-1 protease were weighed higher than others and therefore assigned a weighing factor of 2; mutations L101I/V, I13V, K20R, M46I/L and L90M were weighed lower than others and therefore assigned a weighing factor of 0.5; mutations L24I, D30N, I50L/V, I54L, L76V and V82I correlate with increased susceptibility to tipranavir and therefore assigned a weighing factor of -1.

Table 3 provides a list of weighing factors assigned to each mutation. This table can be used to predict whether an HIV-1 is likely to be resistant or partially resistant to tipranavir. Each of the protease mutations listed in Table 3 that is detected is assigned a weighing factor according to Table 3. The weighing factors are then added to get a mutation score the HIV-1. If the mutation score is 4 or more, then the HIV-1 is likely to be resistant or partially resistant to tipranavir treatment (using a 2-fold cutoff), and if the mutation score is less than 4, then the HIV-1 is likely to be sensitive to tipranavir treatment

### 6.4 Example 4: The New Algorithm and Demonstration of its Improved Accuracy

This example demonstrates that the new algorithm constructed in Example 3 has reduced discordance rate and improved accuracy over the old algorithm used in the Kohlbrenner study.

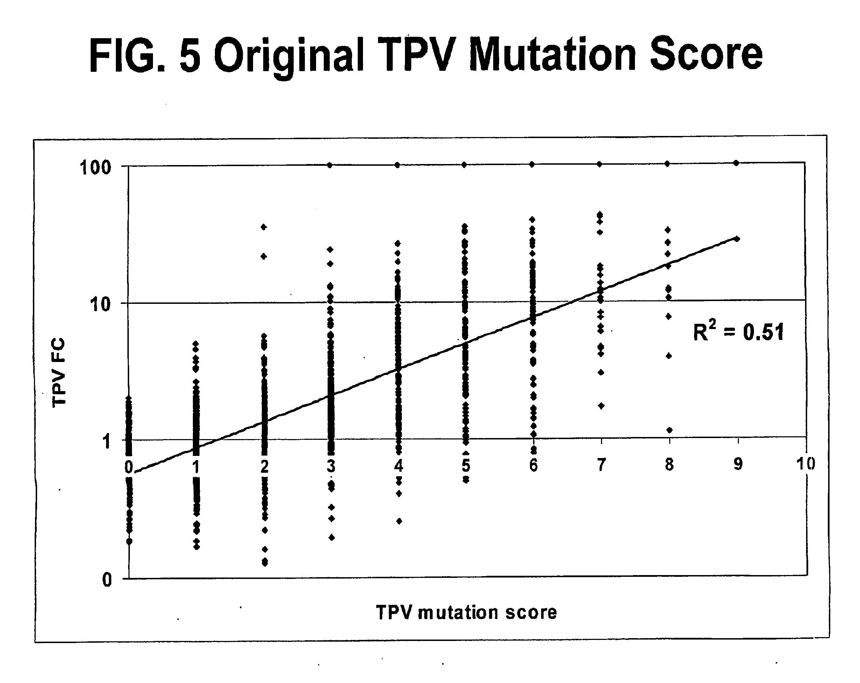

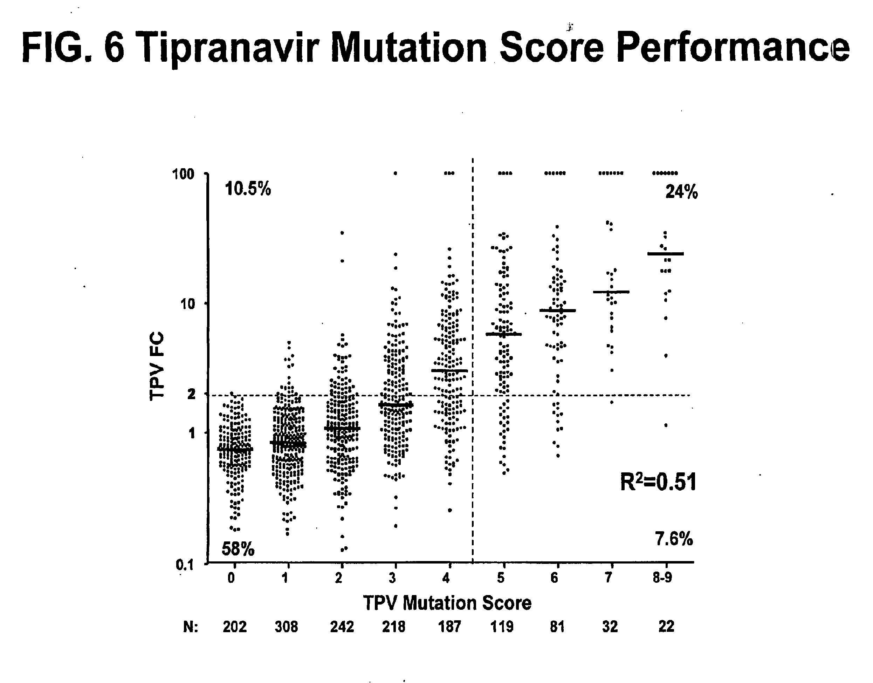

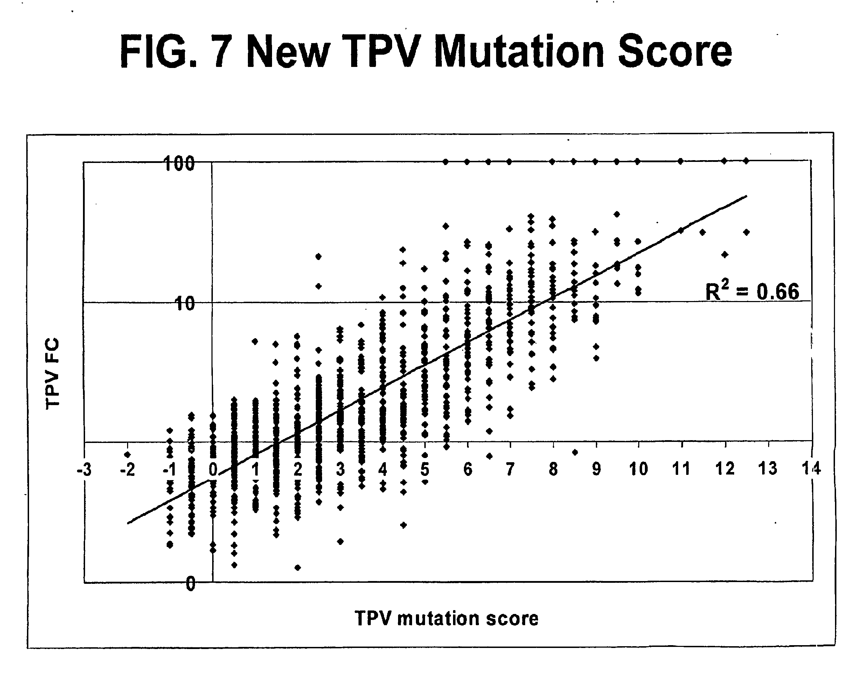

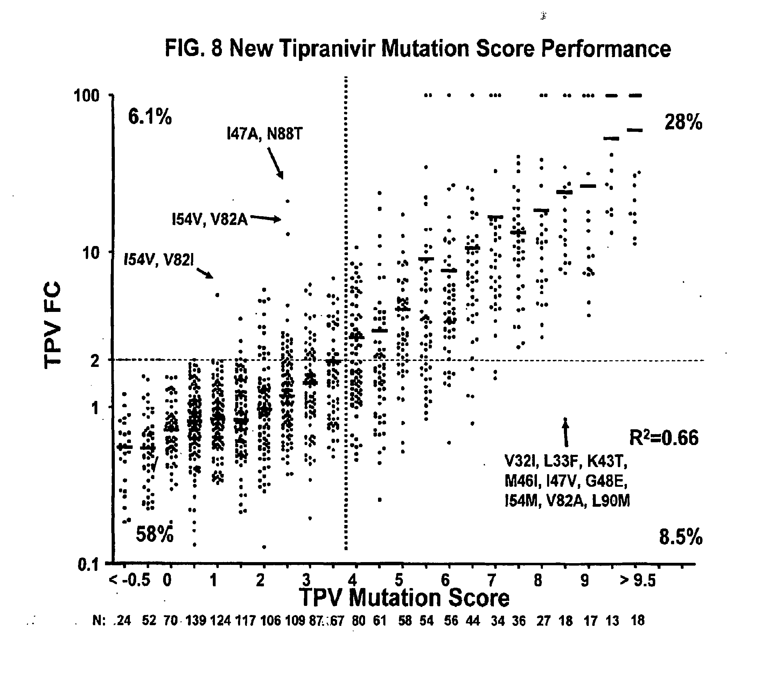

Figs. 5-8 demonstrate that the correlation between tipranavir fold change and mutation score calculated using the new algorithm has a higher linear regression coefficient than that of the Kohlbrenner study. The median tipranavir FC (Log for FC) for each group of samples defined by the mutation score is plotted against the mutation score calculated using the old algorithm (Figs. 5-6) and using the new algorithm (Figs. 7-8). The correlation between tipranavir fold change and mutation score calculated using the old algorithm has a linear regression coefficient of 0.51. *See* Figs. 5-6. The correlation between tipranavir fold change and mutation score calculated using the new algorithm has a linear regression coefficient of 0.66. *See* Figs. 7-8.

A dataset consisting of 1411 patient samples was analyzed genotypically as well as phenotypically, as described in Example 1-3. The weighting factors listed in Table 3 were used to calculate mutation score under the new algorithm. As shown in Table 4, the discordance rate varies depending on different phenotypic and mutation score cutoffs. The optimal cutoff for the mutation score is chosen based on a low discordance rate and balanced distribution of %GrPs (false positive) and % GsPr (false negative).

When the phenotypic cutoff is 2 (tipranavir FC >2), in both the old and new algorithm, the mutation score cutoff was 4, *i.e.,* if the mutation score for a particular HIV was 4 or greater, then the HIV was genotypically resistant or partially resistant to treatment with tipranavir and if the total score was less than 4, then the HIV was genotypically sensitive to treatment with tipranavir. Using a cutoff value of 4, the overall discordance using the new algorithm (14.6%) was lower than that in the old algorithm (18.1%).

When the phenotypic cutoff is 4 (tipranavir FC >4), the optimal mutation score cutoff was changed from 5 for the old algorithm to 6 for the new algorithm. Using the optimal mutation score cutoff, the overall discordance using the new algorithm (10.2%), was also lower than that in the old algorithm (13.8%).

When the phenotypic cutoff is 10 (tipranavir FC >10), the optimal cutoff mutation score was changed from 6 for the old algorithm to 7 for the new algorithm. Using the optimal mutation score cutoff, the overall discordance using the new algorithm , (7.6%) was also lower than that in the old algorithm (9.1%). Thus, the new algorithm taking into account newly-identified mutations associated with altered susceptibility to tipranavir and assigning different weighing factors to certain mutations improves its overall concordance compared with the old algorithm.

### 6.5 Example 5: Application of the New Algorithm to a Naïve Dataset

In this example, the new algorithm, determined as described above, were applied to a naïve dataset to test its predictive power.

The naïve dataset consisted of 1845 patient samples, which contained at least one protease inhibitor resistance mutation (L23X, L24X, D30X, V32X, M46X, I47X, G48X, I50X, I54X, V82A, V82F, V82S, V82T, V82C, V82G, V82L, V82M, I84X, N88X, L90X, where X is any non-wild-type amino acid) and contained no more than one mixture of amino acid residues at positions associated with altered susceptibility to tipranavir listed in Table 3.

The naïve dataset were analyzed genotypically as well as phenotypically as described in Examples 1-3. A mutation score for each sample was calculated according to Example 3 and Table 3 and was used to predict whether the virus in the samples would be susceptible to tipranavir under the new algorithm determined in Example 3. The results of the analysis are presented in Figs. 9-10 and Table 5.

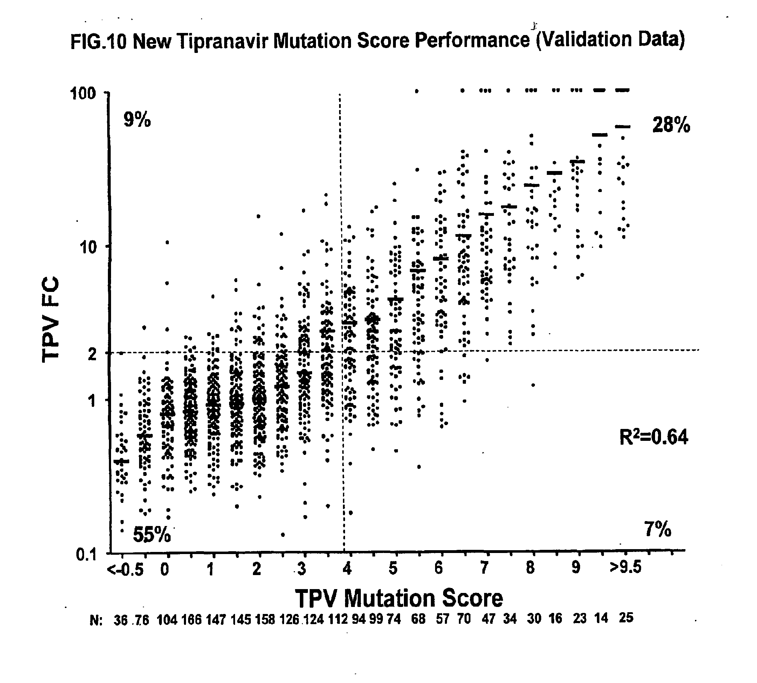

As shown in Fig. 10, when the phenotypic cutoff was selected to be 2 (tipranavir FC >2), and a mutation score cutoff of 4 was used, application of the new algorithm to the naïve dataset identified 55% of samples as Gs-Ps, about 28% of samples as Gr-Pr, about 7% of samples as Gr-Ps and about 9.3% of samples as Gs-Pr. Application of the new algorithm to the naïve dataset correctly predicted the phenotype of 83-84% of samples (1540 out of 1845 samples). The overall discordance rate (Gs-Pr plus Gs-Pr) for the new algorithm was 16.5%. *See* Table 5.

Similarly, as shown in Table 5, when the phenotypic cutoff was selected to be 4 (tipranavir FC >4), and a mutation score cutoff of 6 was used, application of the new algorithm to the naive dataset correctly predicted the phenotype of 89.3% of samples (1647 out of 1845 samples). The overall discordance rate for the new algorithm was 10.7%. When the phenotypic cutoff was selected to be 10 (tipranavir FC >10), and a mutation score cutoff of 4 was used, application of the new algorithm to the naive dataset correctly predicted the phenotype of 92.4% of samples (1708 out of 1845 samples). The overall discordance rate for the new algorithm was 7.6%.

The overall discordance rates for application of the new algorithm to the naïve dataset using different phenotypic and genotypic cutoff are consistent with those for application of the new algorithm to the training dataset (n=1411). *See* Tables 4 and 5.

In addition, the overall discordance rates for application of the new algorithm to the naïve dataset using different phenotypic and genotypic cutoff are lower than those for application of the old algorithm to the naïve dataset. *See* Table 5. For example, when the phenotypic cutoff is 2 and mutation score cutoff was 4, the overall discordance rate using the new algorithm for the naïve dataset was 16.5% and the overall discordance rate using the old algorithm for the naïve dataset was 20.8%.

Further, Fig. 9 and Fig. 10 demonstrate that the correlation between tipranavir fold change and mutation score when the new algorithm was applied to the naïve dataset has a higher linear regression coefficient than that of the old algorithm. The correlation between tipranavir fold change and mutation score when the old algorithm was applied to the naive dataset has a linear regression coefficient of 0.49. *See* Fig. 9. The correlation between tipranavir fold change and mutation score when the new algorithm was applied to the naïve dataset has a linear regression coefficient of 0.64. *See* Fig. 10.

In summary, these results confirm that the new algorithm can predict resistance/susceptibility of an HIV-1 to tipranavir, based on the number of different-weighed mutations in the protease gene of an HIV-1 that were identified as being associated with altered susceptibility to tipranavir. Compared with the old algorithm, the new algorithm has higher overall concordance and higher linear regression coefficient between the phenotypic and genotypic analysis.

### 6.6 Example 6: Construction of another Algorithm from a Larger Dataset

This example describes construction of an additional algorithm for predicting resistance or susceptibility to tipranavir from a larger dataset than the algorithm of Examples 3.5.

First, TPV resistance or suscpetibility phenotype and genotype data were generated according to Example 1. Samples with off-scale TPV resistance (TPV IC₅₀ higher than the highest drug concentration tested in the assay) were assigned a fold-change (FC) value of 100. TPV phenotype data were available for 18,647 samples in this analysis. Of these, 5082 had at least one mutation indicative of prior exposure to PIs, and no more than 1 mixture of wild-type and mutant amino acids at any position included in the TPV mutation score calculated according to the Kohlbrenner study, discussed in Example 1, above. If data derived from more than one sample from the same patient were present, only the most recent data were included. The resulting data set contained 4492 genotype (PR) and phenotype (TPV) data points. In addition, the genotype of the C-terminal 82 amino acids of gag (representing the end of NC/p7, as well as all of p1 and p6) was available for 3880 samples. Half of the samples (2246 for PR, and 1940 for gag) were randomly selected to serve as a training dataset, with the other half acting as a validation dataset.

Samples were grouped based on TPV mutation score calculated according to the Kohlbrenner algorithm of Example 2. The median TPV FC for samples within each group defined by TPV mutation score was calculated, and samples were further categorized based on measured TPV FC being higher (H, FC:median ratio > 1.2) or lower (L, FC:median ratio < 0.8) than the median for each group. Fisher's Exact test with the Benjamini correction for multiple comparisons was used to determine which PR mutations were associated with H (vs. not H) or L (vs. not L) TPV FC. The odds ratio (OR) for each mutation (% of H samples with the mutation to % of L samples with the mutation) was also calculated.

Results from these calculations are summarized in Table 6 and Figure 11. Based on a clinical cut-off of 2-fold, the majority of samples had reduced TPV susceptibility when the TPV mutation score calculated according to the Kohlbrenner algorithm was 4 or greater. *See* Figure 11. While the mean and median FC for each group of samples increased progressively as mutation score increased, and a significant linear correlation was evident between log-transformed FC and mutation score (R²=0.50), extensive variability in FC was evident within any group of samples, and was greatest (%CV > 100) in samples with 1 to 5 TPV mutations. Thus, in these samples, which have FC values close to the clinically relevant threshold, the likelihood of making an accurate prediction about TPV susceptibility based on mutation score was low. For example, among samples with 3 TPV mutations and thus considered sensitive by genotype, 44% had TPV FC greater than 2-fold, and 19% greater than 4-fold, Therefore, these results support the earlier conclusions of Example 2 that the algorithm of the Kohlbrenner study poorly predicts TPV resistance or susceptibility.

To explore mutational correlates associated with high or low TPV FC within groups of samples defined by TPV mutation score calculated according to the Kohlbrenner algorithm, each sample's FC was converted to a ratio relative to the median FC of that group. The relative FC for a randomly selected half of the samples were then grouped together, and mutations in PR or the C-terminal end of gag that are over-represented in samples with FC higher (H, ratio > 1.2) or lower (L, ratio < 0.8) than the median were identified using Fisher's Exact test with correction for multiple comparisons as described above. Table 7 lists the mutations that showed significant associations with H or L TPV FC. Several mutations already in the TPV mutation score were found to be associated with H (I47V, I54A or M, V82T, I84V) or L (L10V, I13V, K20M or R, M36I, M46L, H69K) FC. Recognized primary PI resistance mutations not included in the existing mutation score were also identified, including V32I, M46I, V82F, L90M (H), L24I, D30N, G48V, I50L or V, I54L, and V82A (L).

In addition, less commonly recognized mutations in PR, and in gag, were present in significantly numbers of samples with H or L TPV FC, as shown in Table 7. In gag, mutations of interest include 1437V, P453L, and insertions near the PTAPP motif. Although rare (9 to 11 samples each), L33LL (an insertion of leucine at position 33) and I85L in PR, and Q430R and S498A in gag, were particularly strongly associated with samples having higher than expected TPV FC (OR > 10). The Q430R mutation in gag is located at the p7NC/p1 cleavage site and has previously been reported to occur in PI-resistant HIV.

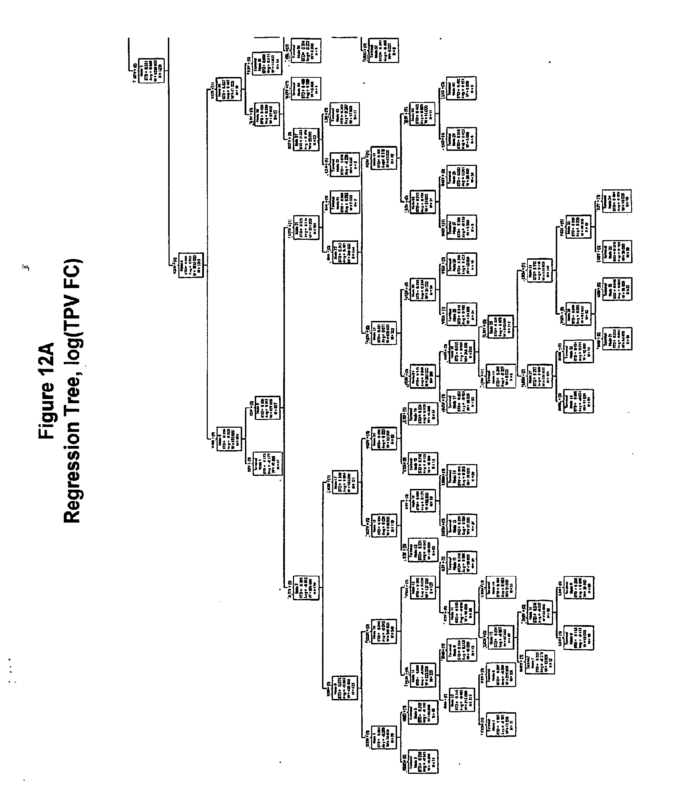

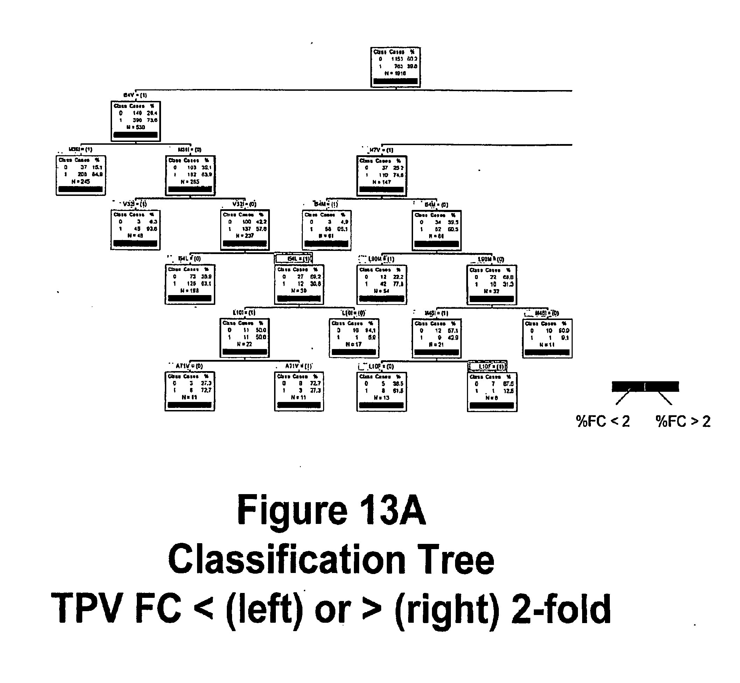

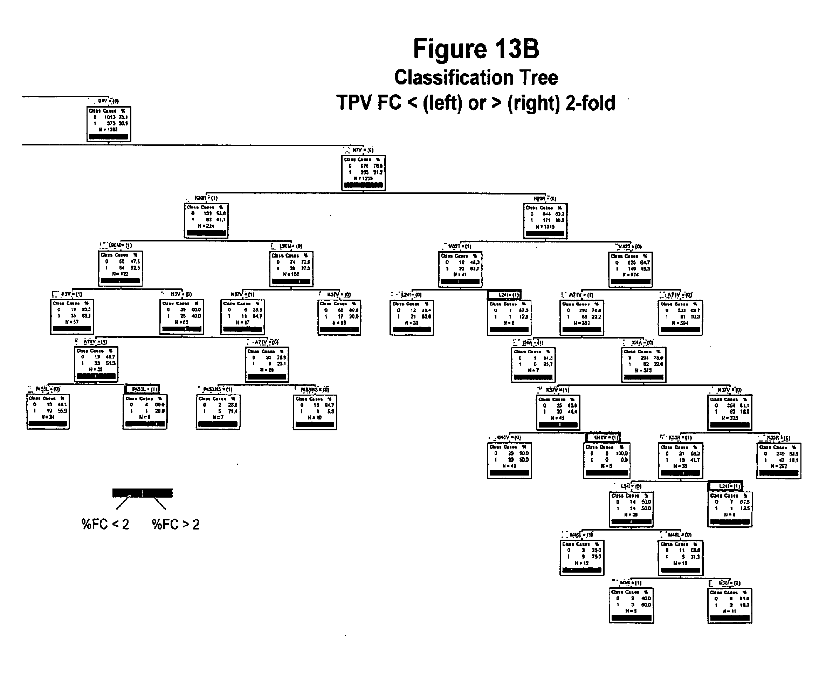

In addition, the prediction power of the proportion of samples with a given mutation was tested in a regression tree (see Figure 12A-C) and a classification tree (see Figure 13A-B) using CART 5.0 software (Salford Systems, San Diego CA). These procedures help identify dichotomous or continuous variables with the greatest ability to separate samples into two groups based on TPV FC. For example, for the regression tree, the presence of I84V was the best separator variable in this analysis, followed by I47V and I13V (See Figure 12A-C). For the classification tree, the the presence of I84V was the best separator variable in this analysis, followed by I47V and M36I (See Figure 13A-B). Results of the classification and regression analysis are also summarized in Table 8, below.

Based on the findings described above, an adjusted algorithm for assessing TPV resistance or susceptibility was constructed by selecting the mutations with the strongest significant associations and adjusting the score assigned to them. The weightings assigned to particular mutations are summarized in Table 9, below. In brief, mutations I47V, I54A, I54M, V82T, I84V were given a score of 2 each and mutations L10V, I13V, K20R, M46L were assigned a score of 0.5. In addition, mutations were added with a score of 1 (V11L, K20M/V, V32I, L33F, E35G, M36I, M36L, K43T, I54M/V, Q58E, H69K, A71L, G73T, T74P, V82L, N83D, L89V in protease and I437V in gag), 0.5 (L10I, M46I, L90M), or -1 (L24I, D30N, G48V, I50L/V, I54L, L76V, V82I), depending on the strength of association. Mutations In some cases, mutations were not included because of the likelihood of strong linkage to other mutations in the new score, such as N88D (often found in samples with D30N) and I85V (often found with I84V or L90M).

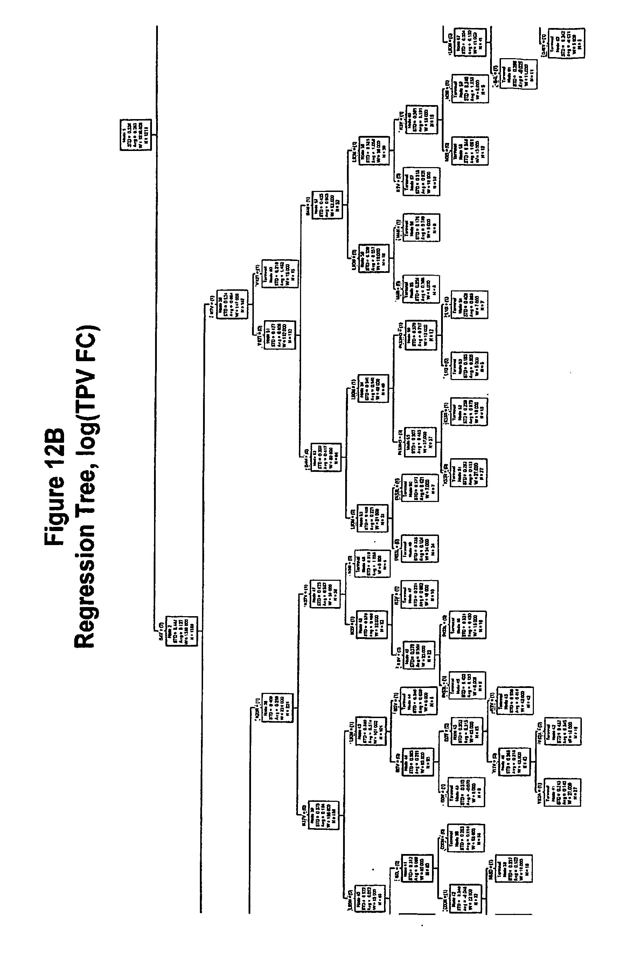

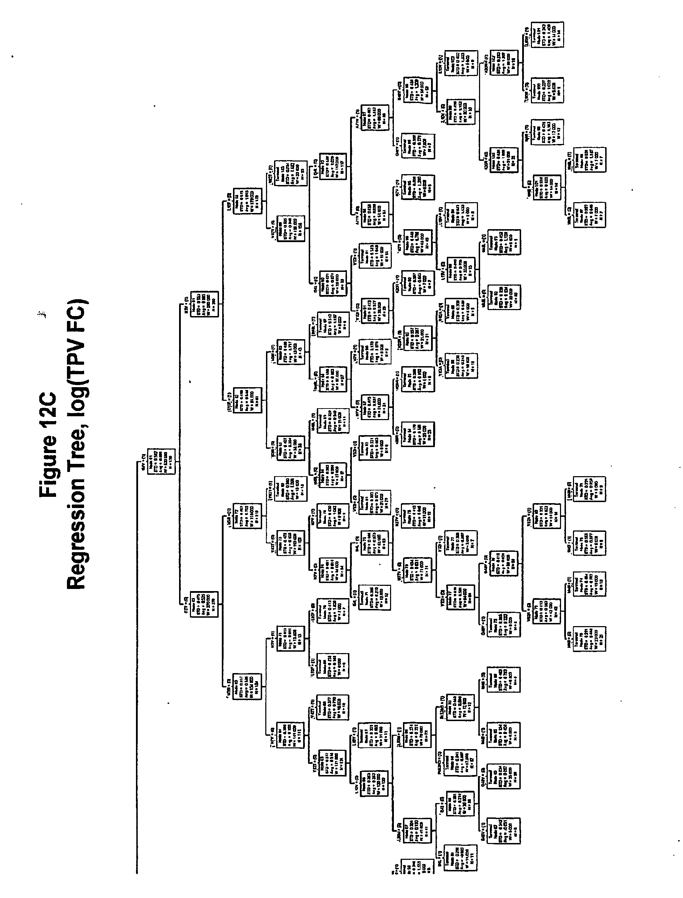

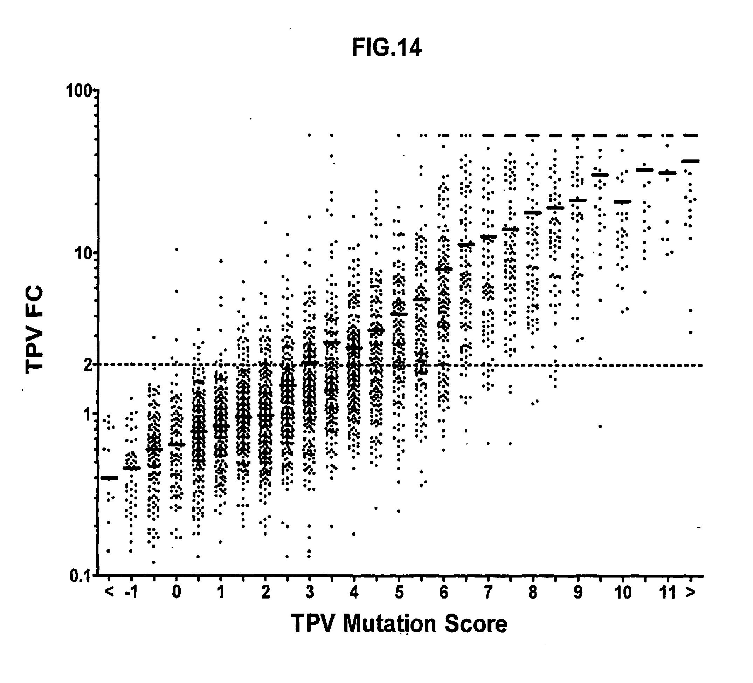

The performance of the adjusted TPV algorithm was tested using the data set used to derive the adjusted mutation score as well as the second half of the starting data set (validation data) by comparing the correlation coefficients derived from scatter plots shown in Figure 11 (calculated using the Kohlbrenner algorithm) and in Figure 14 (calculated using the adjusted TPV algorithm), and the phenotype-genotype discordance rates using a FC value of 2.0 to define phenotypic susceptibility (Table 10). Based on the scatter plots shown in Figures 11 and 12, the correlation between TPV FC and mutation score was significantly improved, from 0.50 to 0.64.

The performance of the old Kohlbrenner algorithm and the adjusted algorithm presented in Table 9 is shown in Tables 10-16. Table 10 shows the concordance of empiricly determined phenotype of the traning data set with phenotype predicted by the old algorithm as 77.6%. Table 11 sorts the training data set by mutation score calculated by the adjusted algorithm, while Table 12 shows the concordance of observed phenotype with predicted genotype as 81.7%

Tables 13-16 apply the old and adjusted algorithms to the validation data set. As shown in Table 14, the overall concordance for predicted versus observed phenotype for the old algorthim was 78.2%, while the corresponding concordance for the adjusted algorithm was 83.3%.

**TABLE 1**

| **Mutation Score** | | | | | | | | | |
|---|---|---|---|---|---|---|---|---|---|
| | **TPV MUTATION SCORE** | | | | | | | | |
| | **0** | **1** | **2** | **3** | **4** | **5** | **6** | **7** | **8-9** |
| **COUNT** | 202 | 308 | 242 | 218 | 187 | 119 | 81 | 32 | 22 |
| **MEAN** | 0.8 | 1.0 | 1.5 | 3.1 | 6.1 | 11 | 16 | 32 | 44 |
| **MEDIAN** | 0.7 | 0.8 | 1.1 | 1.6 | 3.0 | 5.7 | 8.8 | 12 | 24 |
| **SD** | 0.4 | . 0.6 | 2.7 | 7.2 | 13 | 19 | 25 | 38 | 40 |
| **%CV** | 45% | 65% | 175% | 233% | 210% | 165% | 152% | 116% | 91% |

**TABLE 2**

| **Fisher Exact Test Results** | | | |
|---|---|---|---|
| **mutation** | **n mut** | **OR=H/L** | **P-Value** |
| I54A | 16 | 15.1 | 0.00253 |
| A71L | 18 | 8.0 | 0.00497 |
| V11L | 20 | 4.0 | 0.03667 |
| V82T | 65 | 2.8 | 0.00076 |
| I47V | 122 | 2.8 | <0.00001 |
| I47 | 133 | 2.7 | <0.00001 |
| G73T | 66 | 2.5 | 0.00329 |
| L89V | 105 | 2.3 | 0.00034 |
| I84V | 356 | 2.2 | <0.00001 |
| I84 | 361 | 2.2 | <0.00001 |
| V32 | 175 | 2.0 | 0.00006 |
| V32I | 169 | 2.0 | 0.00008 |
| M36L | 77 | 2.0 | 0.02024 |
| I66 | 94 | 1.9 | 0.01722 |
| L89 | 186 | 1.7 | 0.00272 |
| D60E | 217 | 1.6 | 0.00265 |
| K55 | 188 | 1.6 | 0.00680 |
| K55R | 169 | 1.6 | 0.01546 |
| D60 | 234 | 1.5 | 0.00701 |
| L90M | 787 | 1.3 | <0.00001 |
| L90 | 793 | 1.3 | <0.00001 |
| M46I | 495 | 1.3 | 0.00424 |
| L10I | 625 | 1.2 | 0.02199 |
| I13 | 577 | 0.8 | 0.03217 |
| M36I | 549 | 0.8 | 0.03953 |
| I13V | 564 | 0.8 | 0.01566 |
| L10F | 281 | 0.7 | 0.01981 |
| K20R | 234 | 0.7 | 0.04161 |
| L89 | 186 | 1.7 | 0.00272 |
| D60E | 217 | 1.6 | 0.00265 |
| K55 | 188 | 1.6 | 0.00680 |
| K55R | 169 | 1.6 | 0.01546 |
| D60 | 234 | 1.5 | 0.00701 |
| L90M | 787 | 1.3 | <0.00001 |
| L90 | 793 | 1.3 | <0.00001 |
| M46I | 495 | 1.3 | 0.00424 |
| L10I | 625 | 1.2 | 0.02199 |
| I13 | 577 | 0.8 | 0.03217 |
| L10V | 145 | 0.7 | 0.04127 |
| M46L | 201 | 0.6 | 0.00259 |
| N88 | 187 | 0.5 | 0.00007 |
| I54L | 133 | 0.5 | 0.00063 |
| N88D | 153 | 0.5 | 0.00005 |
| V82I | 46 | 0.4 | 0.04848 |
| | | | |

| **mutation** | **n mut** | **R/S** | **P-Value** |
|---|---|---|---|
| L24 | 118 | 0.4 | 0.00004 |
| 150L | 34 | 0.4 | 0.04168 |
| L76 | 49 | 0.3 | 0.00316 |
| L76V | 49 | 0.3 | 0.00303 |
| L24I | 93 | 0.3 | <0.00001 |
| D30 | 142 | 0.2 | <0.00001 |
| D30N | 134 | 0.2 | <0.00001 |
| I50 | 90 | 0.2 | <0.00001 |
| I50V | 53 | 0.1 | <0.00001 |

**TABLE 3**

| **Weighting Factors** | | | |
|---|---|---|---|
| **Mutation** | **Weighting Factor** | **Mutation** | **Weighting Factor** |
| L10I/V | 0.5 | I54A | 2 |
| VIIL | 1 | I54M/V | 1 |
| I13V | 0.5 | I54L | -1 |
| K20M/V | 1 | Q58E | 1 |
| K20R | 0.5 | H69K | 1 |
| L24I | -1 | A71L | 1 |
| D30N | -1 | G73T | 1 |
| V32I | 1 | T74P | 1 |
| L33F | 1 | L76V | -1 |
| E35G | 1 | V82I | -1 |
| M36I | 1 | V82T | 2 |
| M36L | 1 | V82L | 1 |
| K43T | 1 | N83D | 1 |
| M46I/L | 0.5 | I84V | 2 |
| I47V | 2 | L89V | 1 |
| I50L/V | -1 | L90M | 0.5 |

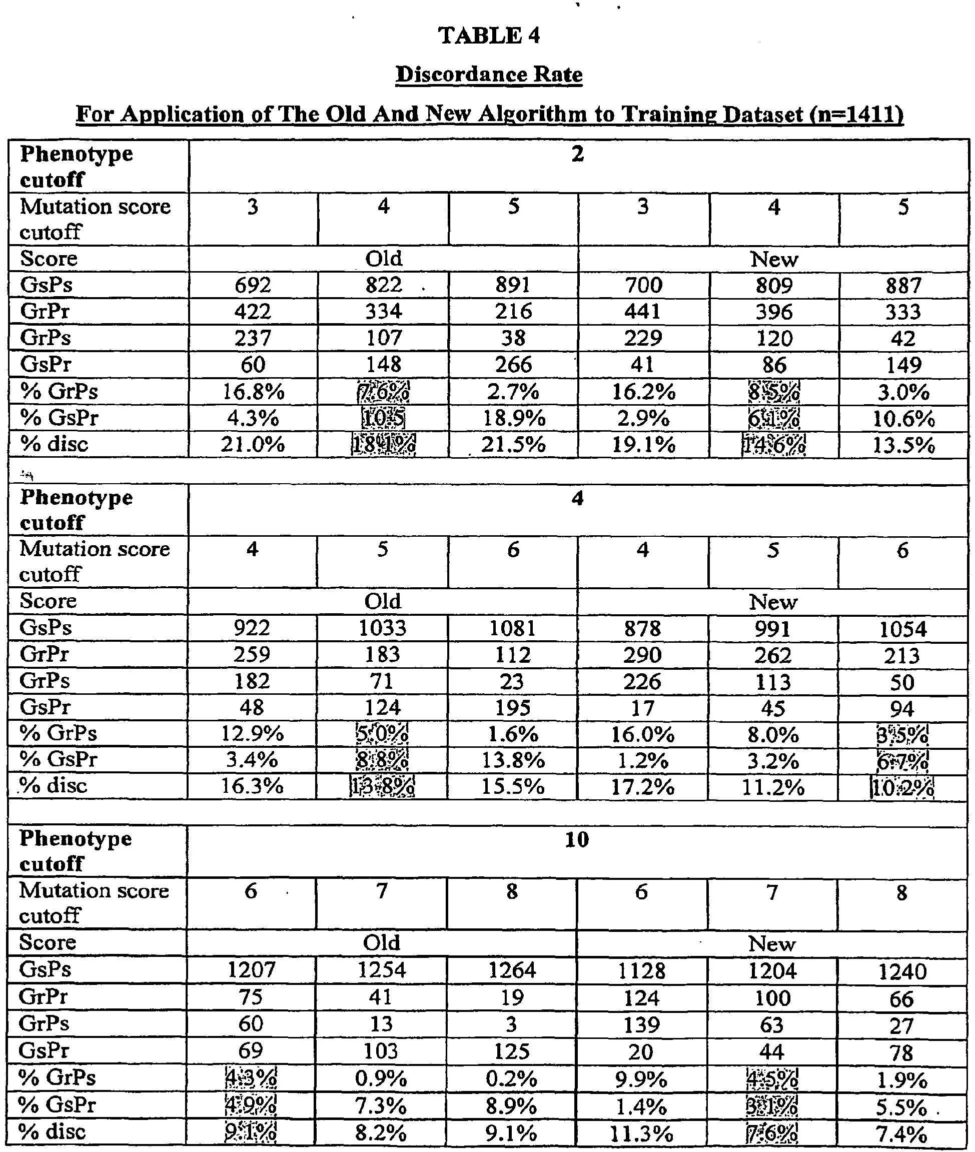

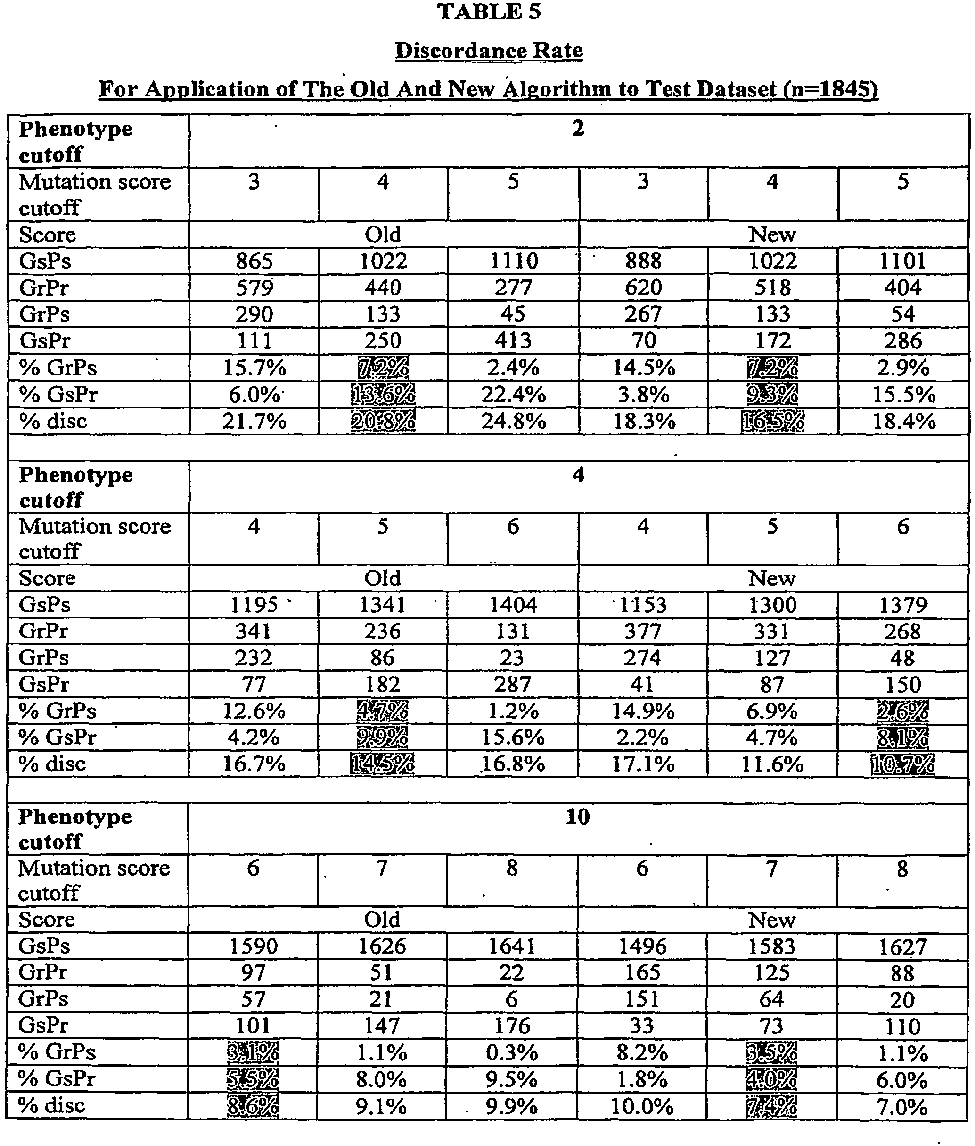

**TABLE 6**

| | TPV Mutation Score | | | | | | | | | |
|---|---|---|---|---|---|---|---|---|---|---|
| | 0 | 1 | 2 | 3 | 4 | 5 | 6 | 7 | 8 | 9 |
| count | 439 | 888 | 793 | 781 | 661 | 443 | 290 | 118 | 62 | 17 |
| Mean FC | 0.8 | 1.1 | 1.6 | 3.1 | 5.8 | 10 | 17 | 24 | 30 | 42 |
| Median FC | 0.7 | 0.9 | 1.1 | 1.7 | 2.9 | 5.5 | 10.7 | 16 | 31 | 53 |
| SD | 0.4 | 2.0 | 2.0 | 4.6 | 9 | 12 | 16 | 19 | 20 | 14 |
| %CV | 55% | 173% | 124% | 150% | 148% | 123% | 99% | 82% | 66% | 34% |

**TABLE 7**

| **protein** | **mutation** | **n mut** | **% mut H^{a}** | **% mut L^{b}** | **OR^{c}** | **p value** |
|---|---|---|---|---|---|---|
| gag | S498A | 11 | 1.4% | 0.1% | 15.93 | 0.01039 |
| PR | L33LL^{d} | 10 | 1.0% | 0.1% | 13.92 | 0.02207 |
| gag | Q430R | 9 | 1.1% | 0.1% | 12.74 | 0.04352 |
| PR | I85L | 9 | 0.9% | 0.1% | 12.37 | 0.04434 |
| PR | L10R | 12 | 1.1% | 0.1% | 7.73 | 0.03471 |
| PR | A71L | 20 | 1.8% | 0.3% | 6.19 | 0.00554 |
| PR | K55N | 25 | 2.3% | 0.4% | 6.19 | 0.00099 |
| PR | E34D | 30 | 2.6% | 0.5% | 5.08 | 0.00099 |
| PR | V82F | 41 | 3.5% | 0.7% | 4.79 | <0.0001 |
| PR | I54A^{e} | 26 | 2.2% | 0.5% | 4.20 | 0.01451 |
| gag | Q500K | 25 | 2.4% | 0.6% | 4.10 | 0.01367 |
| gag | A431I | 32 | 3.1% | 0.8% | 4.07 | 0.00345 |
| PR | V11L | 48 | 3.7% | 1.1% | 3.40 | 0.00099 |
| PR | G73T | 120 | 9.0% | 3.0% | 2.98 | <0.0001 |
| PR | V82T^{e} | 139 | 14% | 3.7% | 2.75 | <0.0001 |
| PR | 147V^{e} | 235 | 16% | 7.1% | 2.20 | <0.0001 |
| PR | I84V^{e} | 633 | 41% | 20% | 2.09 | <0.0001 |
| PR | I54M^{e} | 167 | 11% | 5.2% | 2.09 | <0.0001 |
| PR | V32I | 291 | 18% | 9.6% | 1.89 | <0.0001 |
| PR | I85V | 187 | 12% | 6.2% | 1.86 | 0.00035 |
| gag | P453ins | 104 | 7.4% | 4.2% | 1.79 | 0.04134 |
| PR | M46I | 874 | 50% | 32% | 1.55 | <0.0001 |
| gag | I437V | 257 | 17% | 11% | 1.48 | 0.01367 |
| PR | K55R | 333 | 18% | 13% | 1.40 | 0.01749 |
| gag | P453L | 582 | 35% | 28% | 1.27 | 0.01367 |
| PR | L90M | 1304 | 67% | 53% | 1.27 | <0.0001 |
| PR | L10I | 1052 | 52% | 43% | 1.21 | 0.00099 |
| PR | I13V^{e} | 933 | 38% | 47% | 0.80 | 0.00018 |
| PR | M36I^{e} | 926 | 37% | 48% | 0.78 | <0.0001 |
| PR | L10F | 463 | 18% | 24% | 0.76 | 0.01813 |
| PR | V82A | 775 | 30% | 41% | 0.74 | <0.0001 |
| gag | S498L | 235 | 10% | 15% | 0.66 | 0.01602 |
| PR | K20R^{e} | 451 | 16% | 27% | 0.59 | <0.0001 |
| PR | M46L^{e} | 355 | 12% | 21% | 0.59 | <0.0001 |
| PR | L10V^{e} | 273 | 9.5% | 16% | 0.59 | 0.00010 |
| PR | N88D | 271 | 9.5% | 16% | 0.59 | 0.00010 |
| PR | K45R | 108 | 3.6% | 6.7% | 0.55 | 0.02813 |
| PR | V82I | 94 | 3.1% | 6.0% | 0.51 | 0.02114 |
| PR | I54L | 239 | 7.6% | 15% | 0.50 | <0.0001 |
| PR | L76V | 75 | 2.3% | 5.0% | 0.45 | 0.00970 |
| PR | L23I | 63 | 1.8% | 4.4% | 0.42 | 0.01204 |
| PR | D30N | 264 | 7.6% | 18% | 0.41 | <0.0001 |
| PR | K20M^{e} | 78 | 2.2% | 5.5% | 0.40 | 0.00119 |
| PR | G48V | 111 | 2.9% | 8.1% | 0.36 | <0.0001 |
| PR | H69K^{e} | 34 | 0.9% | 2.5% | 0.35 | 0.03889 |
| PR | L24I | 185 | 4.7% | 14% | 0.34 | <0.0001 |
| PR | I50L | 75 | 1.7% | 6.0% | 0.28 | <0.0001 |
| PR | I50V | 83 | 1.0% | 7.9% | 0.13 | <0.0001 |

**TABLE 8**

| **MUTATION IMPORTANCE** | | | |
|---|---|---|---|
| **MUTATION** | **+ or -** | **REGRESSION** | **CLASSIFICATION** |
| I84V | + | 100.00 | 100.00 |
| I47V | + | 50.46 | 51.02 |
| V32I | + | 36.56 | 43.48 |
| I54M | + | 34.24 | 32.84 |
| M36I | + | 25.28 | 6.20 |
| V82T | + | 24.25 | 8.75 |
| V11L | + | 23.53 | 10.13 |
| I13V | + | 21.83 | 4.01 |
| K20R | + | 14.94 | 18.32 |
| I437V | + | 14.78 | 9.32 |
| M46L | + | 13.17 | 6.53 |
| L10F | - | 11.19 | 2.86 |
| A431I | + | 10.70 | 5.81 |
| D30N | - | 10.35 | 6.88 |
| L90M | + | 10.00 | 9.61 |
| I54A | + | 9.73 | 3.52 |
| A71V | +/- | 8.45 | 12.57 |
| G73T | + | 7.77 | 5.11 |
| I50V | - | 7.52 | 2.23 |
| A71L | + | 6.83 | 3.24 |
| N88D | + | 6.38 | 2.98 |
| L10I | + | 6.07 | 3.65 |
| I54L | - | 5.59 | 4.08 |
| I85L | + | 5.57 | 0.00 |
| K55N | + | 5.13 | 0.00 |
| V82A | + | 5.05 | 4.49 |
| L10V | + | 4.87 | 0.00 |
| Q430R | + | 4.38 | 0.00 |
| L24I | - | 4.17 | 3.76 |
| G48V | - | 4.01 | 1.66 |
| V82F | + | 3.87 | 0.00 |
| I85V | + | 3.54 | 0.00 |
| K55R | + | 3.27 | 3.90 |
| M46I | + | 3.17 | 6.01 |
| P453L | +/- | 2.95 | 9.90 |
| S498L | + | 2.38 | 0.53 |
| L76V | - | 2.08 | 0.05 |
| P453INS | + | 1.97 | 3.22 |
| K45R | + | 1.77 | 1.29 |
| V82I | - | 1.62 | 0.57 |
| Q500K | + | 1.18 | 0.00 |
| L23I | + | 1.16 | 0.00 |
| E34D | + | 1.07 | 0.00 |
| I50L | - | 0.98 | 0.05 |
| L10R | + | 0.75 | 0.00 |
| H69K | + | 0.53 | 0.00 |
| S498A | + | 0.46 | 0.00 |
| K20M | + | 0.27 | 0.05 |
| L33LL | + | 0.01 | 0.00 |

**TABLE 9**

| **Weighting Factors** | | | |
|---|---|---|---|
| **Mutation** | **Weighting Factor** | **Mutation** | **Weighting Factor** |
| L10I/V | 0.5 | I54A | 2 |
| V11L | 1 | I54V | 1 |
| 113V | 0.5 | I54M | 2 |
| K20M/V | 1 | I54L | -1 |
| K20R | 0.5 | Q58E | 1 |
| L24I | -1 | H69K | 1 |
| D30N | -1 | A71L | 1 |
| V32I | 1 | G73T | 1 |
| L33F | 1 | T74P | 1 |
| E35G | 1 | L76V | -1 |
| M36I | 1 | V82I | -1 |
| M36L | 1 | V82T | 2 |
| K43T | 1 | V82L | 1 |
| M46I/L | 0.5 | N83D | 1 |
| I47V | 2 | I84V | 2 |
| G48V | -1 | L89V | 1 |
| I50L/V | -1 | L90M | 0.5 |
| gag I437V | 1 | | |

**TABLE 10**

| Concordance for old mutation score, training data (n=2246) | | | | |
|---|---|---|---|---|
| GROUP | TPV FC | MTN SCORE | N | % |
| PR,GR | > 2 | ≥ 4 | 602 | 26.8% |
| PR,GS | > 2 | < 4 | 318 | 14.2% |
| PS,GR | < 2 | ≥ 4 | 186 | 8.3% |
| PS,GS | < 2 | < 4 | 1140 | 50.8% |
| | (overall concordance: 77.6%) | | | |

**TABLE 11**

| Adjusted Mutation Score, training data with gag (n=1916) | | | | | | | | | | |
|---|---|---|---|---|---|---|---|---|---|---|
| | TPV Mutation Score | | | | | | | | | |
| | <3 | 3 | 3.5 | 4 | 4.5 | 5 | 5.5 | 6 | 6.5 | >=7 |
| count | 876 | 125 | 116 | 129 | 109 | 81 | 71 | 81 | 49 | 279 |
| mean | 1.1 | 2.3 | 3.2 | 2.5 | 3.1 | 4.0 | 5.1 | 7.2 | 10.0 | 19.7 |
| median | 0.9 | 1.5 | 1.5 | 2.0 | 2.2 | 2.8 | 2.4 | 4.9 | 6.3 | 13.4 |
| SD | 1.0 | 4.8 | 6.6 | 2.3 | 3.0 | 3.4 | 7.7 | 8.6 | 12.0 | 17.2 |
| %CV | 94% | 207% | 206% | 89% | 98% | 84% | 152% | 119% | 120% | 87% |

**TABLE 12**

| Concordance for mutation score calculated with adjusted algorithm, training data with gag | | | | |
|---|---|---|---|---|
| GROUP | TPV FC | MTN SCORE | N | % |
| PR,GR | > 2 | ≥ 4 | 605 | 31.6% |
| PR,GS | > 2 | < 4 | 158 | 8.2% |
| PS,GR | < 2 | ≥ 4 | 194 | 10.1% |
| PS,GS | < 2 | < 4 | 959 | 50.1% |
| | (overall concordance: 81.7%) | | | |

**TABLE 13**

| Base mutation score, validation data (n=2246) | | | | | | | | | | |
|---|---|---|---|---|---|---|---|---|---|---|
| | TPV Mutation Score | | | | | | | | | |
| | 0 | 1 | 2 | 3 | 4 | 5 | 6 | 7 | 8 | 9 |
| count | 221 | 451 | 418 | 384 | 342 | 227 | 117 | 55 | 29 | 2 |
| mean | 0.8 | 1.1 | 1.7 | 3.4 | 6.4 | 10.7 | 18 | 24 | 31 | 41 |
| median | 0.7 | 0.9 | 1.2 | 1.8 | 3.2 | 5.7 | 11.7 | 19 | 33 | 41 |
| SD | 0.5 | 1.8 | 2.2 | 4.4 | 9.5 | 13 | 17 | 20 | 20 | 18 |
| %CV | 57% | 155% | 126% | 133% | 149% | 121% | 95% | 81% | 65% | 43% |

**TABLE 14**

| Concordance for old mutation score, validation data (n=2246) | | | | |
|---|---|---|---|---|
| GROUP | TPV FC | MTN SCORE | n | % |
| PR,GR | > 2 | ≥ 4 | 597 | 26.6% |
| PR,GS | > 2 | < 4 | 314 | 14.0% |
| PS,GR | < 2 | ≥ 4 | 175 | 7.8% |
| PS,GS | < 2 | < 4 | 1160 | 51.6% |

| | | | | |
|---|---|---|---|---|
| (overall concordance: 78.2%) | | | | |

**TABLE 15**

| Adjusted Mutation Score, validation data with gag (n=1916) | | | | | | | | | | |
|---|---|---|---|---|---|---|---|---|---|---|
| | TPV Mutation Score | | | | | | | | | |
| | <3 | 3 | 3.5 | 4 | 4.5 | 5 | 5.5 | 6 | 6.5 | >=7 |
| count | 849 | 129 | 112 | 115 | 95 | 89 | 96 | 101 | 66 | 264 |
| mean | 1.1 | 1.8 | 2.3 | 2.6 | 3.5 | 4.4 | 5.2 | 8.6 | 12.6 | 20.1 |
| median | 0.9 | 1.4 | 1.5 | 2.0 | 2.4 | 2.8 | 3.2 | 4.5 | 7.4 | 12.5 |
| SD | 0.9 | 1.9 | 2.9 | 2.0 | 4.1 | 6.1 | 6.6 | 10.4 | 11.4 | 17.5 |
| %CV | 87% | 103% | 123% | 76% | 116% | 139% | 127% | 121% | 90% | 87% |

**TABLE 16**

| Concordance for mutation score calculated with adjusted algorithm, validation data with gag | | | | |
|---|---|---|---|---|
| GROUP | TPV FC | MTN SCORE | n | % |
| PR,GR | > 2 | ≥ 4 | 641 | 33.5% |
| PR,GS | > 2 | < 4 | 136 | 7.1% |
| PS,GR | < 2 | ≥ 4 | 185 | 9.7% |
| PS,GS | < 2 | < 4 | 954 | 49.8% |

| | | | | |
|---|---|---|---|---|
| (overall concordance: 83.3%) | | | | |

## Claims

1. A method for determining whether a human immunodeficiency virus type 1 (HIV-1) is likely to have a reduced susceptibility to tipranavir, comprising:
(a) detecting, in a gene encoding a protease of the HIV-1, the presence or absence of HIV-1 protease mutations, each of said mutations being associated with a weighting factor;
and
(b) summing the weighting factors for each of the mutations that are detected in step (a) to compute a mutation score for the HIV- 1;
**wherein the method is characterized by**
detecting the presence or absence of the HIV-1 protease mutations listed in Table 3 or 9;
each of said mutations being associated with a weighting factor as shown in Table 3 or 9;
wherein the HIV-1 is likely to have a reduced susceptibility to tipranavir if said mutation score is equal to or greater than 4;
wherein the HIV-1 is an HIV-1 from a patient sample.

2. The method of claim 1, wherein the HIV-1 is an HIV-1 isolated from a patient sample.

3. The method of claim 1, wherein the HIV-1 is likely to have a reduced susceptibility to tipranavir if said mutation score is equal to or greater than 5, equal to or greater than 6, or equal to or greater than 7.

4. The method of claim 1, wherein the HIV-1 is isolated from the patient sample without passage through cell culture.

5. A computer implemented method of determining whether an HIV-1 is likely to have a reduced susceptibility to tipranavir, comprising:
(a) inputting to a computer-readable medium genotypic data from an HIV-1 protease of the HIV-1;
(b) comparing the genotypic data to a database that comprises the set of mutations listed in table 3 or 9, each of said mutations being associated with a weighting factor as shown in Table 3 or 9; and
(c) calculating a mutation score by summing the weighting factors associated with the mutation(s) present in the genotype of the HIV-1;
wherein if the mutation score is equal to or greater than 4, the HIV-1 is determined to have a reduced susceptibility to tipranavir.

6. A computer-readable medium comprising a computer program that determines whether an HIV-1 infecting a subject is likely to have a reduced susceptibility to tipranavir, comprising:
(a) a computer code that receives input corresponding to a genotype of a HIV-1 nucleic acid encoding HIV-1 protease obtained from HIV-1 infecting the subject;
(b) a computer code that performs a comparison to determine if the mutations listed in Table 3 or 9 are present or absent,
(c) a computer code that calculates a mutation score based on the number of mutations present;
(d) a computer code that determines whether the mutation score is equal to or greater than a cutoff score, wherein the HIV-1 is determined to have a reduced susceptibility to tipranavir if the mutation score is equal to or greater than 4; and
(e) a computer code that conveys a result representing whether or not the HIV-1 is determined to have a reduced susceptibility to tipranavir to an output device.

7. The computer-readable medium of claim 6, wherein the medium is selected from the group comprising a floppy disk, CD, DVD, magnetic tape, and fixed disk drive.

## Patentansprüche

1. Verfahren zur Bestimmung, ob ein humanes Immundeffizienzvirus Typ I (HIV-1) voraussichtlich eine reduzierte Empfindlichkeit gegenüber Tipranavir hat, umfassend:
(a) Detektion in einem Gen, kodierend für eine Protease des HIV-1 die Anwesenheit oder die Abwesenheit von HIV-1 Proteasemutationen, wo jede von diesen Mutationen mit einem Gewichtsfaktor assoziiert ist; und
(b) Summieren der Gewichtsfaktoren für jede der Mutationen, die in Schritt (a) nachgewiesen sind, um eine Mutationszahl für das HIV-1 zu errechnen;
wobei das Verfahren durch folgendes gekennzeichnet ist:
Detektion der Anwesenheit oder der Abwesenheit der in Tabellen 3 und 9 aufgelisteten HIV-1 Proteasemutationen;
jede der Mutationen ist mit einem Gewichtsfaktor gemäß Tabellen 3 und 9 assoziiert;
wobei das HIV-1 voraussichtlich eine reduzierte Empfindlichkeit gegenüber Tipranavir hat, wenn die Mutationszahl gleich oder größer als 4 ist;
wobei das HIV-1 ist ein HIV-1 aus einer Patientenprobe ist.

2. Das Verfahren nach Anspruch 1, wobei das HIV-1 ein HIV-1 ist, das aus einer Patientenprobe isoliert ist.

3. Das Verfahren nach Anspruch 1, wobei der HIV-1 voraussichtlich eine reduzierte Empfindlichkeit gegenüber Tipranavir hat, wenn die Mutationszahl gleich oder größer als 5, gleich oder größer als 5 oder gleich oder größer als 7 ist.

4. Das Verfahren nach Anspruch 1, wobei der HIV-1 aus der Patientenprobe ohne eine Passage durch eine Zellkultur isoliert ist.

5. Ein computerimplementiertes Verfahren zur Bestimmung, ob ein HIV-1 voraussichtlich eine reduzierte Empfindlichkeit gegenüber Tipranavir hat, umfassend:
(a) Eingabe auf ein computerlesbares Medium von genotypischen Daten aus einer HIV-1 Protease aus dem HIV-1;
(b) Vergleich der genotypischen Daten mit einer Datenbank, umfassend den Satz der in Tabellen 3 und 9 aufgelisteten Mutationen, wobei jede der Mutationen mit einem Gewichtsfaktor gemäß Tabelle 3 oder 9 assoziiert ist; und
(c) Ausrechnen einer Mutationszahl durch Summieren der Gewichtsfaktoren, die mit der(n) Mutation(en), die in dem Genotyp des HIV-1 vorhanden sind, assoziiert sind;
wobei wenn die Mutationszahl gleich oder größer als 4 ist, das HIV-1 bestimmt als solches mit reduzierter Empfindlichkeit gegenüber Tipranavir wird.

6. Ein computerlesbares Medium, umfassend ein Computerprogramm, das bestimmt, ob ein HIV-1, das ein Subjekt infiziert, voraussichtlich eine reduzierte Empfindlichkeit gegenüber Tipranavir hat, umfassend:
(a) ein Computercode, der ein Input entsprechend einem Genotyp einer HIV-1 Nukleinsäure bekommt, kodierend für HIV-1 Protease, die aus dem HIV-1 isoliert wurde, das das Individuum infiziert hat;
(b) ein Computercode, der einen Vergleich durchführt um zu bestimmen, ob die in Tabellen 3 und 9 aufgelisteten Mutationen anwesend oder abwesend sind;
(c) ein Computercode, der eine Mutationszahl kalkuliert, basierend auf der Anzahl von vorhandenen Mutationen;
(d) ein Computercode, der bestimmt, ob die Mutationszahl gleich oder größer als eine Cutoff-Zahl ist, wobei das HIV-1 als solches mit einer reduzierten Empfindlichkeit gegenüber Tipranavir bestimmt wird, wenn die Mutationszahl gleich oder größer ist als 4; und
(e) ein Computercode, der ein Ergebnis, das zeigt, ob das HIV-1 bestimmt ist, eine reduzierte Empfindlichkeit gegenüber Tipranavir zu haben, zu einem Ausgabegerät weiterleitet.

7. Das computerlesbares Medium nach Anspruch 6, wobei das Medium ausgewählt ist aus der Gruppe, umfassend eine Diskette, CD, DVD, Magnetband, und ein festes Laufwerk.

## Revendications

1. Procédé permettant de déterminer s'il y a des chances pour qu'un virus d'immuno-déficience humaine de type 1 (VIH-1) ait une sensibilité réduite au tipranavir, comprenant :
(a) la détection, dans un gène codant pour une protéase du VIH-1, de la présence ou de l'absence de mutations de la protéase du VIH-1, chacune desdites mutations étant associée à un facteur de pondération ; et
(b) l'addition des facteurs de pondération pour chacune des mutations qui sont détectées dans l'étape (a) pour calculer un score de mutation pour le VIH-1 ;
ledit procédé étant **caractérisé par**
la détection de la présence ou de l'absence des mutations de la protéase du VIH-1 énumérées dans le tableau 3 ou le tableau 9 ;
chacune desdites mutations étant associée à un facteur de pondération comme indiqué dans le tableau 3 ou le tableau 9 ;
ledit VIH-1 ayant des chances d'avoir une sensibilité réduite au tipranavir si ledit score de mutation est égal ou supérieur à 4 ;
ledit VIH-1 étant un VIH-1 provenant d'un échantillon de patient.

2. Procédé selon la revendication 1, dans lequel le VIH-1 est un VIH-1 isolé à partir d'un échantillon provenant de patient.

3. Procédé selon la revendication 1, dans lequel il y a des chances pour que le VIH-1 ait une sensibilité réduite au tipranavir si ledit score de mutation est égal ou supérieur à 5, égal ou supérieur à 6, ou bien égal ou supérieur à 7.

4. Procédé selon la revendication 1, dans lequel le VIH-1 est isolé à partir de l'échantillon provenant d'un patient sans passage par une culture de cellules.

5. Procédé de mise en oeuvre informatique permettant de déterminer s'il y a des chances pour qu'un VIH-1 ait une sensibilité réduite au tipranavir, comprenant :
(a) l'entrée, sur un support pouvant être lu par un ordinateur, de données génotypiques provenant d'une protéase de VIH-1 du VIH-1 ;
(b) la comparaison des données génotypiques à une base de données qui comprend l'ensemble des mutations énumérées dans le tableau 3 ou le tableau 9, chacune desdites mutations étant associée à un facteur de pondération comme indiqué dans le tableau 3 ou le tableau 9 ; et
(c) le calcul d'un score de mutation en additionnant les facteurs de pondération associés à la/les mutation(s) présente(s) dans le génotype du VIH-1 ;
dans lequel, si le score de mutation est égal ou supérieur à 4, il est établi que le VIH-1 a une sensibilité réduite au tipranavir.

6. Support pouvant être lu par un ordinateur comprenant un programme d'ordinateur qui détermine s'il y a des chances pour qu'un VIH-1 infectant un sujet ait une sensibilité réduite au tipranavir, comprenant :
(a) un code d'ordinateur qui reçoit une entrée correspondant à un génotype d'un acide nucléique de VIH-1 codant pour la protéase du VIH-1 obtenue à partir du VIH-1 infectant le suj et ;
(b) un code d'ordinateur qui effectue une comparaison afin de déterminer si les mutations énumérées dans le tableau 3 ou le tableau 9 sont présentes ou absentes ;
(c) un code d'ordinateur qui calcule un score de mutation fondé sur le nombre de mutations présentes ;
(d) un code d'ordinateur qui détermine si le score de mutation est égal ou supérieur à un score limite, le VIH-1 étant considéré comme ayant une sensibilité réduite au tipranavir si le score de mutation est égal ou supérieur à 4 ; et
(e) un code d'ordinateur qui transmet à un dispositif de sortie un résultat exprimant s'il est établi ou non que le VIH-1 a une sensibilité réduite au tipranavir.

7. Support pouvant être lu par un ordinateur selon la revendication 6, ledit support étant choisi dans le groupe comprenant une disquette, un CD, un DVD, une bande magnétique et un lecteur de disques fixe.
